(19) Europäisches Patentamt
European Patent Office
Office européen des brevets

(11) Veröffentlichungsnummer: **0 222 703**
**A1**

(12) **EUROPÄISCHE PATENTANMELDUNG**

(21) Anmeldenummer: **86810496.9**

(22) Anmeldetag: **31.10.86**

(51) Int. Cl.$^4$: **C 07 D 493/04**
C 07 D 491/052,
C 07 D 493/14,
C 07 D 491/153, A 61 K 31/44
//(C07D493/04,311:00,221:00),
(C07D491/052,335:00,221:00)

(30) Priorität: **08.11.85 US 796348**

(43) Veröffentlichungstag der Anmeldung:
**20.05.87 Patentblatt 87/21**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Schneider, Josef A.**
**99 Main Street**
**Millburn New Jersey 07041 (US)**

(54) **Hexahydro-[1]-benzo-(pyrano und thiopyrano)-[4,3-c]pyridine.**

(57) Die Erfindung betrifft

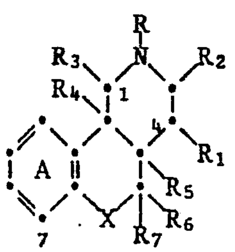

(I),

worin X Sauerstoff oder Schwefel bedeutet, der Ring A unsubstituiert oder durch Hydroxy, veräthertes Hydroxy, Acyloxy. Halogen, Niederalkyl, Arylniederalkyl oder Trifluormethyl ein- bis dreifach oder durch Alkylendioxy an zwei nachbarständigen C-Atomen substituiert sein kann, R Wasserstoff, Niederalkyl, Niederalkenyl, Niederalkinyl, Aroylniederalkyl oder Arylniederalkyl, $R_1$ Wasserstoff, Niederalkyl, Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl, $R_2$ - $R_5$ Wasserstoff oder Niederalkyl und $R_6$ und $R_7$ Wasserstoff bedeuten oder Niederalkyl bedeuten mit der Massgabe, dass $R_1$ Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl bedeuten, und Salze, insbesondere pharmazeutisch annehmbare Salze, dieser Verbindungen.

Diese Verbindungen sind als Serotonin-2-Rezeptor-Antagonisten bei der Behandlung von psychotropen Störungen wie Angstzuständen, Depressionen und Manie verwendbar.

EP 0 222 703 A1

**Beschreibung**

Hexahydro-[1]-benzo-(pyrano und thiopyrano)-[4,3-c]pyridine

Gegenstand der vorliegenden Erfindung sind neue Hexahydro-2H-[1]-benzopyrano- und benzothiopyrano[4,3-c]pyridine, welche als Serotoninrezeptor-Antagonisten wirksam sind, Verfahren zu ihrer Herstellung, pharmazeutische Zusammensetzungen und Behandlungsverfahren von Zuständen, Syndromen und Krankheiten durch Anwendung der neuen Verbindungen oder von pharmazeutischen Zusammensetzungen mit diesen Verbindungen.

Die vorliegende Erfindung betrifft insbesondere neue Hexahydro-2H-[1]-benzopyrano[4,3-c]pyridin-Derivate und neue Hexahydro-2H-[1]-benzothiopyrano[4,3-c]-Derivate der allgemeinen Formel

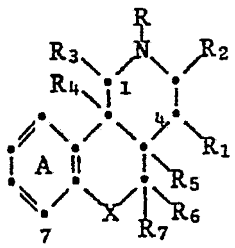

(I),

worin X Sauerstoff oder Schwefel bedeutet, der Ring A unsubstituiert oder durch Hydroxy, veräthertes Hydroxy, Acyloxy, Halogen, Niederalkyl, Arylniederalkyl oder Trifluormethyl ein- bis dreifach oder durch Alkylendioxy an zwei nachbarständigen C-Atomen substituiert sein kann, R Wasserstoff, Niederalkyl, Niederalkenyl, Niederalkinyl, Aroylniederalkyl oder Arylniederalkyl, $R_1$ Wasserstoff, Niederalkyl, Niederalkylthio-niederalkyl, Arylniederalkylthio-niederalkyl, Aminoniederalkyl, Niederalkylaminoniederalkyl, Diniederalkylamino niederalkyl, Acylaminoniederalkyl, Hydroxyniederalkyl, Acyloxyniederalkyl, veräthertes Hydroxyniederalkyl oder Cyanniederalkyl, $R_2$ - $R_5$ Wasserstoff oder Niederalkyl und $R_6$ und $R_7$ Wasserstoff bedeuten oder Niederalkyl bedeuten mit der Massgabe, dass $R_1$ Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl bedeuten, und Salze, insbesondere pharmazeutisch annehmbare Salze, dieser Verbindungen.

Die weiter vorn und im folgenden genannten allgemeinen Begriffe und Definitionen haben im Rahmen der Beschreibung der Erfindung vorzugsweise die folgenden Bedeutungen:

Der im Zusammenhang mit organischen Resten und Gruppen, z.B. Niederalkyl, Niederalkoxy, Niederalkanoyl etc. verwendete Ausdruck "Nieder" bedeutet, dass solche organischen Reste und Gruppen, falls nicht ausdrücklich anders definiert, bis einschliesslich 7 und bevorzugt bis einschliesslich 4 Kohlenstoffatome enthalten.

Bevorzugt sind Verbindungen der Formel I, worin X, der Ring A, R, $R_1$ und $R_2$ - $R_5$ die weiter vorn genannten Bedeutungen haben und $R_6$ und $R_7$ Wasserstoff bedeuten.

Weiterhin sind Verbindungen der Formel I bevorzugt, worin $R_2$ - $R_7$ Wasserstoff bedeuten.

Ebenfalls bevorzugt sind Verbindungen der Formel I, worin X Sauerstoff oder Schwefel bedeutet, der Ring A unsubstituiert oder durch Hydroxy, Acyloxy, veräthertes Hydroxy, Arylniederalkyl, Niederalkyl oder Halogen ein- bis dreifach oder durch Alkylendioxy an zwei nachbarständigen C-Atomen substituiert sein kann, R Wasserstoff oder Niederalkyl, $R_1$ Wasserstoff, Niederalkyl, Niederalkylthio-niederalkyl oder Niederalkoxyniederalkyl und $R_2$ - $R_7$ Wasserstoff bedeuten, und pharmazeutisch annehmbare Salze dieser Verbindungen.

Bevorzugt sind weiterhin Verbindungen der Formel I, worin der Ring A unsubstituiert ist oder durch Niederalkyl, Aryl-niederalkyl, Niederalkoxy, Aryl-niederalkoxy oder Halogen ein- bis dreifach substituiert sein kann, X Sauerstoff oder Schwefel, R Wasserstoff oder Niederalkyl, $R_1$ Wasserstoff, Niederalkyl, Niederalkylthioniederalkyl oder Niederalkoxymethyl und $R_2$ - $R_7$ Wasserstoff bedeuten, und pharmazeutisch annehmbare Salze dieser Verbindungen.

Besonders bevorzugt sind Verbindungen der Formel I, worin X Sauerstoff oder Schwefel bedeutet, der Ring A durch $C_1$-$C_4$-Alkoxy, Benzyloxy, $C_1$-$C_4$-Alkyl oder Halogen ein- bis dreifach substituiert sein kann, R $C_1$-$C_4$-Alkyl, $R_1$ Wasserstoff, $C_1$-$C_4$-Alkyl, $C_1$-$C_4$-Alkylthiomethyl oder $C_1$-$C_4$-Alkoxymethyl und $R_2$ - $R_7$ Wasserstoff bedeuten, und pharmazeutisch annehmbare Salze dieser Verbindungen.

Bevorzugter Erfindungsgegenstand sind ausserdem Verbindungen der Formel I, worin X Sauerstoff bedeutet.

Die Verbindungen der Formel I haben cis- oder trans-Ringverknüpfung. In Abhängigkeit von der Bedeutung von $R_1$ - $R_7$ und der daraus sich ergebenden Anzahl an chiralen C-Atomen können die Verbindungen der Formel I in Form von Racematen, Diastereomeren oder optisch reinen Antipoden vorliegen.

Aufgrund der cis-, trans-Ringverknüpfung liegen die Verbindungen der Formel I ausserdem als geometrische Isomere vor. Sämtliche isomeren Formen, welche von der allgemeinen Formel I umfasst werden, sind ebenfalls Gegenstand der vorliegenden Erfindung.

Bevorzugt sind Verbindungen der Formel I mit einer trans-4a,10b-Ringverknüpfung. Daraus resultieren

[4aS*,10bR*]- oder [4aR*,10bS*]-Verbindungen.

Ganz besonders bevorzugt sind Verbindungen der allgemeinen Formel

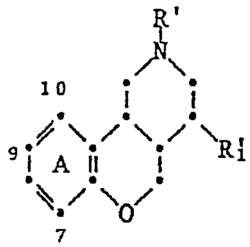

(II),

vorzugsweise mit trans-4a,10b-Ringverknüpfung, worin der Ring A vorzugsweise in 9- oder 10-Stellung durch $C_1$-$C_4$-Alkoxy und in 7-Stellung durch $C_1$-$C_4$-Alkyl oder Halogen substituiert ist, R' $C_1$-$C_4$-Alkyl und $R^o_1$ Wasserstoff oder $C_1$-$C_4$-Alkylthiomethyl bedeuten, und pharmazeutisch annehmbare Salze dieser Verbindungen.

Ganz besonders sind Verbindungen der Formel II bevorzugt, worin trans-4a,10b-Ringverknüpfung vorliegt und der Ring A in 7-Stellung durch $C_1$-$C_3$-Alkyl, Brom oder Chlor und in 9-Stellung durch $C_1$-$C_3$-Alkoxy substituiert ist, R' $C_1$-$C_3$-Alkyl und $R^o_1$ Wasserstoff oder $C_1$-$C_3$-Alkylthiomethyl bedeuten, und pharmazeutisch annehmbare Salze davon.

Niederalkyl ist keradkettig oder - falls möglich - verzweigt und enthält vorzugsweise 1 bis 4 C-Atome, z.B. Aethyl, Propyl oder n-Butyl, insbesondere Methyl.

Niederalkoxy ist geradkettig oder - falls möglich - verzweigt und enthält vorzugsweise 1 bis 4 C-Atome, z.B. Methoxy, Aethoxy oder Propoxy.

Niederalkenyl enthält vorzugsweise 3 oder 4 C-Atome und ist z.B. Allyl oder Crotonyl.

Niederalkinyl enthält vorzugsweise 3 oder 4 C-Atome und ist z.B. Propargyl.

Niederalkylthio ist geradkettig oder - falls möglich - verzweigt und ist z.B. Methylthio, Aethylthio, n-Propylthio oder n-Butylthio.

Niederalkanoyl ist z.B. Acetyl, Propionyl oder n-Butyryl.

Aroyl ist vorzugsweise Benzoyl oder Benzoyl ein- bis dreifach substituiert durch Niederalkyl, Niederalkoxy, Halogen oder Trifluormethyl.

Heteroaroyl ist vorzugsweise Thienoyl, Pyrroloyl oder 2-, 3- oder 4-Pyridylcarbonyl, insbesondere Nicotinoyl.

Aryl, z.B. der Arylrest in Arylniederalkyl, ist vorzugsweise Phenyl, 1- oder 2-Naphthyl oder Phenyl ein- bis dreifach substituiert durch Niederalkyl, Niederalkoxy, Halogen oder Trifluormethyl.

Arylniederalkyl ist insbesondere Benzyl oder 2-Phenäthyl, welche am Phenylrest durch Niederalkyl, Niederalkoxy, Halogen oder Trifluormethyl ein- bis dreifach substituiert sein können.

Arylniederalkyl ist vorzugsweise 1- oder 2-Naphthylmethyl oder 1-oder 2-Naphthyläthyl.

Niederalkanoyloxy ist vorzugsweise Acetoxy oder Propionyloxy.

Niederalkanoylamino ist vorzugsweise Acetamido oder Propionamido.

Aroyloxy ist vorzugsweise Benzoyloxy oder Benzoyloxy ein- bis dreifach substituiert durch Niederalkyl, Niederalkoxy, Halogen oder Trifluormethyl oder hat die Bedeutung von Heteroaroyloxy.

Heteroaroyloxy ist vorzugsweise 2-, 3- oder 4-Pyridylcarbonyloxy, insbesondere Nicotinoyloxy.

Halogen ist vorzugsweise Brom, kann aber auch Fluor, Chlor oder Jod sein.

Niederalkylendioxy ist vorzugsweise Aethylendioxy oder Methylendioxy.

Veräthertes Hydroxy ist vorzugsweise Niederalkoxy, z.B. Methoxy oder Aethoxy, Niederalkenyloxy, z.B. Allyloxy, Niederalkinyloxy, z.B. Propargyloxy, $C_3$-$C_6$-Cycloalkylniederalkoxy, z.B. Cyclopropylmethoxy, Arylniederalkoxy, z.B. Benzyloxy, oder Benzyloxy ein- bis dreifach substituiert am Phenylring durch Niederalkyl, Niederalkoxy oder Halogen, Pyridyl-$C_1$-$C_4$-alkoxy, z.B. Pyridylmethoxy, Naphthylniederalkoxy, z.B. 1- oder 2-Naphthylmethoxy oder 2-(1-Naphthyl)-äthoxy oder 2-(2-Naphthyl)-äthoxy, Fluorenyloxy, z.B. 9-Fluorenyloxy, oder Fluorenylniederalkoxy, z.B. 1-Fluorenylmethoxy oder 2-(1-Fluorenyl)-äthoxy.

Veräthertes Hydroxy kann ausserdem die Gruppierung der Teilformel

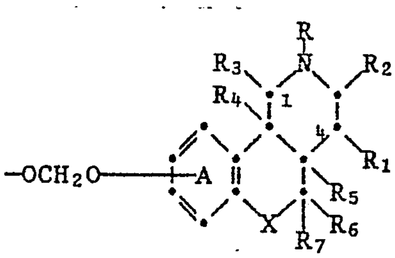

(Ia)

darstellen, worin der Ring A durch weitere Substituenten substituiert sein kann, z.B. durch Halogen, Niederalkyl oder Trifluormethyl, und worin die Reste R, $R_1$ - $R_7$ und X die unter Formel I genannten

Bedeutungen haben. Insbesondere ist die -$OCH_2$-O-Brücke mit der 9-Stellung des tricyclischen Ringsystems verknüpft. Die daraus resultierenden Verbindungen der Formel I, welche durch die weiter vorn genannten verätherten Hydroxygruppen substituiert sind, sind vorzugsweise Bis-Verbindungen der allgemeinen Formel

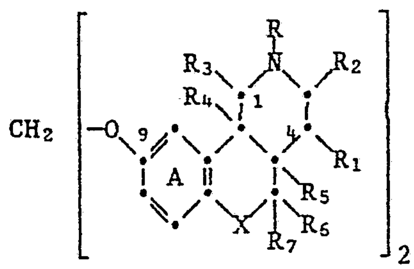

(Ib),

worin der Ring A und die Gruppen X, R und $R_1$ - $R_7$ die weiter vorn genannten Bedeutungen haben.

Niederalkylthio-niederalkyl ist vorzugsweise geradkettiges $C_1$-$C_4$-Alkylthio-$C_1$-$C_4$-alkyl, insbesondere $C_1$-$C_4$-Alkylthiomethyl.

Aryl-niederalkylthio-niederalkyl ist vorzugsweise geradkettiges Aryl-$C_1$-$C_4$-alkylthio-$C_1$-$C_4$-alkyl, z.B. 2-Arylähylthiomethyl, worin Aryl die weiter vorn genannten Bedeutungen hat.

Aminoniederalkyl ist z.B. Aminomethyl oder 2-Aminoäthyl. Niederalkylaminoniederalkyl ist z.B. Methylaminomethyl oder 2-Methylaminoäthyl.

Diniederalkylaminoniederalkyl ist z.B. Dimethylaminomethyl.

Hydroxyniederalkyl ist z.B. Hydroxymethyl oder 2-Hydroxyäthyl.

Acyloxyniederalkyl ist z.B. $C_1$-$C_4$-Alkanoyloxymethyl, z.B. Acetoxymethyl.

Veräthertes Hydroxyniederalkyl ist z.B. $C_1$—$C_4$-Alkoxymethyl, z.B. Methoxy- oder Aethoxymethyl.

Cyanniederalkyl ist insbesondere Cyanmethyl.

Acylamino ist insbesondere Niederalkanoylamino, Arylniederalkanoylamino, Aroylamino, Heteroaroylamino, Niederalkoxycarbonylamino und Benzyloxycarbonylamino mit den für die betreffenden Gruppen weiter vorn genannten Bedeutungen. Acylamino ist ausserdem Phthalimido.

Acyloxy ist insbesondere Niederalkanoyloxy, Aroyloxy sowie Aroyloxy mit den für die betreffenden Gruppen weiter vorn genannten Bedeutungen.

Mono- oder Diniederalkylamino sind insbesondere Mono- oder Di-(methyl, äthyl oder -n-propyl)-amino.

Pharmazeutisch annehmbare Salze sind therapeutisch verwendbare Säureadditionssalze, vorzugsweise Salze von anorganischen oder organischen Säuren, z.B. starken Mineralsäuren, z.B. Halogenwasserstoffsäuren, z.B. Chlorwasserstoff oder Bromwasserstoff, Schwefel-, Phosphor- oder Salpetersäure, aliphatischen oder aromatischen Carbonsäuren oder Sulfonsäuren, z.B. Ameisen-, Essig-, Propion-, Bernstein-, Glycol-, Milch-, Apfel-, Wein-, Zitronen-, Malein-, Fumar-, Brenztrauben-, Phenylessig-, Benzoe-, 4-Aminobenzoe-, Anthranil-, 4-Hydroxybenzoe-, Salicyl-, 4-Aminosalicyl-, Glucon-, Pamoa-, Nicotin-, Methansulfon-, Ethansulfon-, Hydroxyethansulfon-, Benzolsulfon-, p-Toluolsulfon-, Naphthalinsulfon-, Sulfanil-, Cyclohexylsulfamin- oder Ascorbinsäure.

Die neuen Verbindungen der Formel I sind in bekannten In-vivo- und In-vitro-Testanordnungen wirksam, welche eine Wirkungsbeziehung hinsichtlich Wirksamkeit von Serotonin-2-Rezeptor-Antagonisten bei der Behandlung von Störungen des zentralen Nervensystems, der Herz-Kreislaufkrankheiten und des Gastrointestinaltrakts in Säugetieren, vor allem Menschen, herstellen.

Ueberdurchschnittliche Serotonin-2-Rezeptor-Aktivität ist an Störungen des zentralen Nervensystems wie Angstzuständen, Depressionen und Manie, beteiligt, sowie an Störungen des Herz-Kreislaufsystems, z.B. Bluthochdruck, und an gastrointestinalen Störungen, z.B. Magengeschwüren.

Die genannten Eigenschaften lassen sich in in-vitro und in-vivo Versuchen, vorzugsweise bei Säugetieren, z.B. Ratten, Hunden oder Affen, aber auch an entnommenen isolierten Organen, Gewebsproben oder enzymatischen Aufbereitungen nachweisen. Die Verbindungen können in-vitro in Form von Lösungen, z.B. wässrigen Lösungen, und in-vivo entweder enteral oder parenteral, mit Vorteil oral oder intravenös, z.B. in Gelatinekapseln, als Stärkesuspensionen oder in wässrigen Lösungen, appliziert werden. Der Dosisbereich in-vitro kann zwischen $10^{-4}$ und $10^{-9}$ molaren Konzentrationen betragen. Der Dosisbereich in-vivo kann zwischen 0,05 und 30 mg/kg/Tag, vorzugsweise zwischen 0,10 und 1 mg/kg/Tag betragen.

Die Bindungseigenschaften an Serotonin-2 (HT-2) Rezeptoren, welche für die erfindungsgemässen Verbindungen mit Serotonin-2 antagonistischer Wirkung indiziert sind, lassen sich in-vitro durch Bestimmung ihrer Fähigkeit, die spezifische Bindung an $^3$H-Ketanserin nach der von Battaglia et al. in Life Sciences 33, 2011 (1983) beschriebenen Methode oder in Membranzubereitungen von frontaler oder parietaler Cortex aus Sprague-Dawley-Ratten mit 200-225 g Gewicht bestimmen. $IC_{50}$-Werte, welche die Konzentrationen angeben, die eine Testverbindung zur Verdrängung von 50 % von spezifisch gebundenem 0,4 nm $^3$H-Ketanserin benötigt, werden durch Log-Logit-Bestimmung der spezifischen Bindungswerte ermittelt. Die Selektivität an HT-2 Rezeptoren kann ebenfalls durch Bindung an Serotonin-1 (HT-1)-Rezeptoren nach der von Middlemiss D.N. und Fozard J.R. in Eur. J. Pharmacol. 90, 151 ff. (1980) angegebenen Methode bestimmt werden.

Der Serotonin-2 Antagonismus kann in-vivo durch Messung der Hemmung des Kopfzuckens induziert

durch 5-Hydroxytryptophangaben (Vorstufe im Serotoninmetabolismus) in der Ratte bestimmt werden. Dieser Test, der bei der Bestimmung von Serotonin-2-Rezeptoreigenschaften im zentralen Nervensystem verwendet wird, ist in Neuropharmacology, 16, 663 ff. (1977) und im J. Pharmacol. Exp. Ther. 228, 133 (1984) beschrieben und wird wie folgt durchgeführt:

Man lässt männliche Wistar-Ratten (120-180 g) 18 Stunden lang vor Versuchsbeginn fasten (ausreichende Wasserversorgung). Alle Versuchstiere werden mit dem peripheren Decarboxylaseinhibitor Alpha-Methyl-Dopa-hydrazin (Carbidopa 25 mg/kg i.p.) und 30 Minuten später mit 5-Hydroxytryptophan (5 HTP 100 mg/kg s.c., 4,0 ml/kg) vorbehandelt. Man überführt 90 Minuten nach der Verabreichung von 5 HTP die Ratten einzeln in durchsichtige Beobachtungsbehälter und zählt die Anzahl der Kopfzuckungen jedes Tieres in einem Beobachtungszeitraum von 10 Minuten. Die Testverbindung oder die entsprechende Zubereitung werden entweder eine halbe Stunde vor Beginn des Beobachtungszeitraums in einer Dosis von 1,0 ml/kg i.p. oder eine, zwei oder vier Stunden vorher in einer Dosis 10 ml/kg p.o. verabreicht. Die $ED_{50}$-Werte werden mittels Probitanalyse bestimmt.

Die erfindungsgemässen Verbindungen sind im Serotonin-2-Rezeptor-Bindungs-Assay noch bei Konzentrationen von 15 mM wirksam. Im "Head-Twitch"-Test sind sie bei der niedrigen Dosis von 1 mg/kg wirksam. So haben z.B. [4R*,4aR*,10bR*]-7-Brom-4-(äthylthiomethyl)-1,3,4,4a,5,10b-hexahydro -9-methoxy-2-methyl-2H-[1]benzopyrano-[4,3-c]pyridin hydrochlorid einen $IC_{50}$-Wert von 22 mM ($2,2 \times 10^{-8}$ M) im Serotonin-2-Rezeptor-Bindungstest bei der spezifischen Bindung an $^3$H-Ketanserin) und eine $ED_{50}$ von 1,1 mg/kg i.p. (bei 30 Minuten) und 0,71 mg/kg p.o. (bei 1 Stunde) im "Head Twitch" Test.

Weitere Wirkungen der erfindungsgemässen Verbindungen aufgrund ihrer Serotonin-2-Blockadeeigenschaften, z.B. Wirkungen auf das zentrale Nervensystem und das kardiovaskuläre System lassen sich im Tierversuch anhand bekannter Methoden nachweisen. Beispielsweise können anxiolytische Wirkungen im Standard-Cook-Davidson-Konfliktmodell in der Ratte nachgewiesen werden. Antihypertensive Eigenschaften lassen sich bei der spontan hypertensiven Ratte nachweisen.

Die besagten vorteilhaften Befunde belegen die Verwendbarkeit der erfindungsgemässen Verbindungen, insbesondere als Serotonin-2-Rezeptor-Antagonisten, als Heilmittel für Säuger (Menschen und Tiere) bei der Behandlung von psychotropen Störungen wie Angstzuständen, Depressionen und Manie, bei der Behandlung von gastrointestinalen Störungen, wie Magengeschwüren, und bei der Behandlung von kardiovaskulären Störunge wie Bluthochdruck.

Die erfindungsgemässen Verbindungen, welche von den Formeln I und I dargestellt werden, sind nach folgenden Verfahren herstellbar:

a) Reduktion einer Verbindung der Formel:

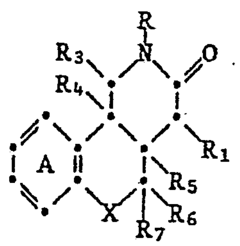

(III),

worin der Ring A, X, R, $R_1$ und $R_3$ - $R_7$ die weiter vorn genannten Bedeutungen haben,

b) durch Reduktion einer ungesättigten Verbindung, welche den Verbindungen der Formel I entspricht, welche aber über eine zusätzliche C-C-Doppelbindung innerhalb von einem oder von beiden heterocyclischen Ringsystemen oder über eine zusätzliche Kohlenstoff-Stickstoff-Doppelbindung (Imin- oder Iminiumbindung) verfügt;

c) durch Zyklisieren einer Verbindung der Formel IV oder IVa

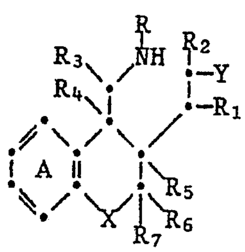

(IV)

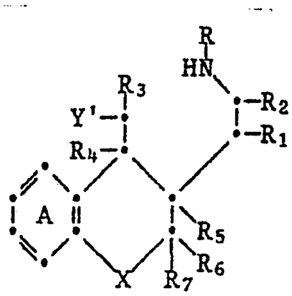

(IVa),

worin der Ring A, X, R und $R_1$ - $R_7$ die genannten Bedeutungen haben und Y und Y' verestertes Hydroxy.

darstellen;

d) durch Reduktion einer Verbindung der Formel

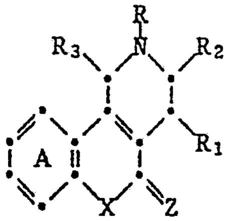

(V)

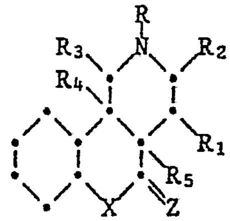

(Va),

worin in beiden Formeln Z Sauerstoff bedeutet, und X, R und $R_1$ - $R_3$ (Formel V) oder X, R und $R_1$ - $R_5$ (Formel Va) wie weiter vorn definiert sind;

e) zur Herstellung einer Verbindung der Formel I, worin $R_6$ und $R_7$ dieselben Bedeutungen haben und Niederalkyl darstellen, eine Verbindung der Formel V oder Va mit einer Metall-Niederalkyl-Verbindung kondensiert und gewünschtenfalls eine eventuelle C-C-Doppelbindung an der Ringverknüpfungsstelle reduziert;

f) zur Herstellung einer Verbindung der Formel I, worin $R_1$ - $R_5$ Wasserstoff sind, die Pyridin- oder Pyridiniumgruppe (wenn R eine andere Bedeutung als Wasserstoff hat) in einer Verbindung der Formel

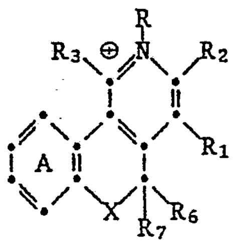

$D^{\ominus}$ (VI),

reduziert, worin der Ring A, X, $R_1$ - $R_3$, $R_6$ und $R_7$ die genannten Bedeutungen haben, und D− das Anion einer organischen oder anorganischen Säure ist;

g) eine Verbindung der Formel

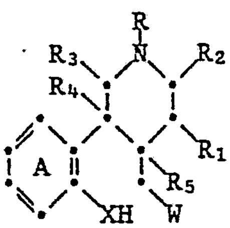

(VII),

worin der Ring A, X, R und $R_1$ - $R_5$ die genannten Bedeutungen haben, und W verestertes Hydroxy darstellt, zyklisiert;

h) zur Herstellung einer Verbindung der Formel I, worin $R_4$ und $R_5$ Wasserstoff sind, eine Verbindung der Formel

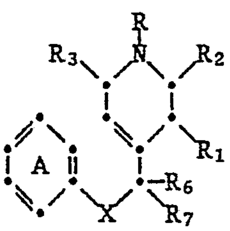

(VIII),

worin der Ring A, X, R, $R_1$ - $R_4$, $R_6$ und $R_7$ die genannten Bedeutungen haben, zyklisiert;

i) zur Herstellung einer Verbindung der Formel I, worin $R_2$ und $R_3$ Wasserstoff sind, eine Verbindung der Formel

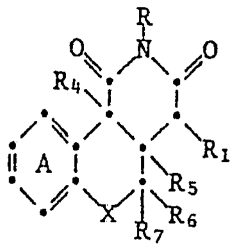

(IX),

worin X, R und $R_1$ und $R_4$ - $R_7$ die weiter vorn genannten Bedeutungen haben oder eine Verbindung der Formel

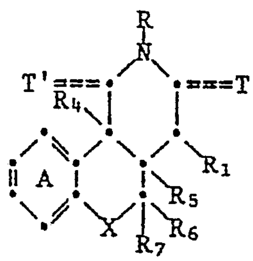

(IXa),

worin T Sauerstoff bedeutet, wenn T' die Bedeutung von zwei Wasserstoffatomen oder von einem Wasserstoffatom und einer Niederalkylgruppe hat, oder worin T' Sauerstoff bedeutet, wenn T die Bedeutung von zwei Wasserstoffatomen oder von einem Wasserstoffatom und einer Niederalkylgruppe hat, und X, R, $R_1$ und $R_4$ - $R_7$ wie oben definiert sind, reduziert;

j) zur Herstellung einer Verbindung der Formel I, worin R Niederalkyl oder Arylniederalkyl bedeutet, eine Verbindung der Formel

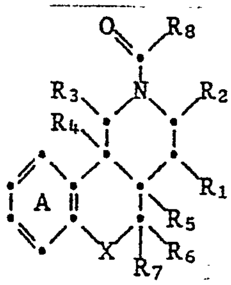

(X),

worin der Ring A, X und $R_1$ - $R_7$ die genannten Bedeutungen haben, $R_8$ Wasserstoff oder Aryl oder Niederalkyl oder Arylniederalkyl mit einer um ein C-Atom verkürzten Kettenlänge bedeutet, reduziert;

k) zur Herstellung einer Verbindung der Formel I, worin $R_4$ - $R_7$ Wasserstoff sind, eine Verbindung der Formel

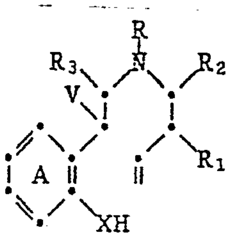

(XI),

worin der Ring A, X, R und $R_1$ - $R_3$ die genannten Bedeutungen haben und V verestertes Hydroxy bedeutet, zyklisiert;

l) eine Verbindung der Formel

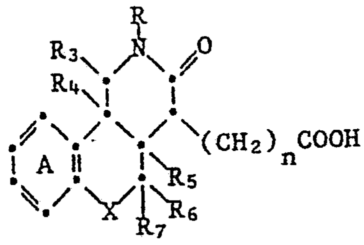

(XII),

oder ein reaktives, funktionelles Derivat davon, z.B. einen Niederalkylester und worin n die Werte null oder eins bis sechs hat und der Ring A, X, R und $R_3$ bis $R_7$ die genannten Bedeutungen haben, durch Decarboxylierung und Reduktion oder durch Reduktion und Umwandlung der Seitenkette derivatisiert, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in einzelne Isomere oder Racemate auftrennt und/oder ein Racemat in die optischen Antipoden aufspaltet.

Eine reaktionsfähige, veresterte Hydroxygruppe in den genannten Verfahrensvarianten ist beispielsweise eine Hydroxygruppe, welche durch eine starke Säure, insbesondere eine starke anorganische Säure wie Halogenwasserstoff, z.B. Chlor-, Brom- oder Jodwasserstoff, oder Schwefelsäure, oder durch eine starke organische Säure, beispielsweise eine Sulfonsäure, wie eine aliphatische oder aromatische Sulfonsäure, z.B. Methansulfonsäure, p-Toluolsulfonsäure oder 4-Brombenzolsulfonsäure, verestert ist. Eine solche reaktionsfähige, veresterte Hydroxygruppe ist insbesondere Halogen, z.B. Chlor, Brom oder Jod, oder durch aliphatische oder aromatische Gruppen substituiertes Sulfonyloxy, z.B. Mesyloxy oder Tosyloxy.

Ein reaktives, funktionelles Derivat einer Carbonsäure, welches bei der Verfahrensvariante 1) verwendet wird, ist z.B. ein gemischtes Anhydrid, z.B. ein Säurehalogenid, z.B. ein Säurechlorid, oder ein Halogenameisensäureester, z.B. Aethylchlorformat, ein reaktiver Ester, z.B. ein Vinylester oder ein Phenylester, z.B. 4-Nitrophenyl-oder 2,4,5-Trichlorphenylester, oder ein reaktives Amid, welches z.B. aus Imidazolderivaten, z.B. N,N-Carbonyldiimidazol, erhältlich ist.

Die in den Ausgangsmaterialien, Zwischenstufen und in Verbindungen der Formel I vorhandenen funktionellen Gruppen, insbesondere die Carboxy-, Amino-, Hydroxy- und Sulfogruppen, sind gegebenenfalls durch solche Schutzgruppen (conventional protecting groups) geschätzt, die üblicherweise in der Penicillin-, Cephalosporin- und Peptidchemie verwendet werden. Diese Schutzgruppen sollen die betreffenden funktionellen Gruppen gegen unerwünschte Nebenreaktionen wie Acylierungen, Verätherungen, Veresterungen, Oxydationen, Solvolyse etc. schützen und unter schonenden Reaktionsbedingungen leicht abspaltbar sein, das heisst unter Vermeidung von Spaltung z.B. solvolytischer, reduktiver, photolytischer oder auch enzymatischer Spaltung, sowie anderer Nebenreaktionen.

Der Schutz von funktionellen Gruppen mit solchen Schutzgruppen, die Schutzgruppen selbst sowie ihre Abspaltungsreaktionen sind beispielsweise in "Protective Groups in Organic Chemistry", Plenum Press, London und New York 1973, in Greene, Th.W. "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I Georg Thieme Verlag, Stuttgart 1974, beschrieben.

Die Herstellung von Verbindungen gemäss Verfahrensvariante a) erfolgt vorzugsweise durch Reduktion mit einem einfachen Hydridreduktionsmittel oder einem solchen komplexen Hydridreduktionsmittel, welches auf die Amidfunktion reduzierend wirkt, z.B. mit Lithiumaluminiumhydrid oder Diboran in einem inerten organischen Lösungsmittel z.B. in Tetrahydrofuran oder Diäthyläther, vorzugsweise bei Raum- oder erhöhter Temperatur.

Verfahren b) wird nach bekannten Methoden zur Reduktion von Doppelbindungen ausgeführt, z.B. mit einem Reduktionsmittel wie Diimid, oder insbesondere zur Herstellung von Verbindungen der Formel I, worin X Sauerstoff bedeutet, mit Wasserstoff unter Hydrierungsbedingungen, vorzugsweise in Gegenwart eines Katalysators wie Palladium auf Kohle, mit Lithium in flüssigem Ammoniak oder, wenn die zu reduzierende Verbindung ein Enamin ist, mit einem Reduktionsmittel wie Natriumcyanborhydrid unter bekannten Reaktionsbedingungen bei Raumtemperatur oder erhöhter Temperatur und zwar in einem polaren organischen Lösungsmittel, z.B. in Isopropanol.

Das Herstellungsverfahren gemäss Verfahrensvariante c) wird vorzugsweise in Gegenwart von basischen Katalysatoren wie Triäthylamin, Natrium- oder Kaliumcarbonat in einem inerten organischen Lösungsmittel analog bekannten N-Alkylierungsreaktionen ausgeführt.

Die Herstellung von Verbindungen der Formel I gemäss Verfahrensvariante d) wird vorzugsweise durch Reduktion mit einem Hydridreduktionsmittel durchgeführt, insbesondere mit einem komplexen Hydridreduktionsmittel in Gegenwart einer bekannten Lewis-Säure, z.B. mit Lithiumaluminiumhydrid oder Natriumborhydrid in Gegenwart von Bortrifluorid oder Aluminiumchlorid. Wenn eine Kohlensteoff-Kohlenstoff-Doppelbindung reduziert werden soll, kann das wie unter Verfahren b) beschrieben geschehen.

Die Herstellung von Verbindungen der Formel I gemäss Verfahrensvariante e) erfolgt vorzugsweise durch Kondensation mit einem Niederalkylmagnesiumhalogenid analog einer Grignard-Reaktion und zwar in einem inerten, wasserfreien Lösungsmittel, z.B. einer Mischung aus Diäthyläther und Anisol, bei einer Temperatur von Raumtemperatur bis zum Siedepunkt des Reaktionsgemisches. Die gegebenenfalls nötige Hydrierung der Doppelbindung kann analog Verfahrensvariante b) erfolgen.

Die Verfahrensvariante f) ist Methoden analog, welche für die Reduktion von Pyridinen und Pyridinium-Verbindungen bekannt sind, z.B. bei solchen Ausgangsmaterialien, worin X Sauerstoff bedeutet, durch katalytische Hydrierung oder durch Reduktion mit reduzierenden Hydriden oder Komplexhydriden, z.B. Natriumnborhydrid oder Aluminiumhydrid. Man erhält Verbindungen mit 4a,10b-Doppelbindung, welche man seinerseits analog Verfahrensvariante b) reduzieren kann.

Die Herstellung von erfindungsgemässen Verbindungen der Formel I analog Verfahrensvariante g) erfolgt auf an sich bekannt Weise üblicherweise in einem Lösungsmittel oder Lösungsmittelgemisch. Wenn nötig wird gekühlt oder erwärmt, z.B. in einem Temperaturbereich von ca. 20° bis 150°C und gegebenenfalls unter Schutzgas, z.B. unter Stickstoff. Das Herstellungsverfahren erfolgt üblicherweise in Gegenwart einer Base, z.B. einer anorganischen Base, z.B. einem Alkalimetall- oder Erdalkalimetallcarbonat, -hydrid oder -hydroxid, oder in Gegenwart einer organischen Base wie einem Niederalkanolat, z.B. Natriumäthanolat, oder einem tertiären Amin wie Triäthylamin oder Pyridin.

Ringschlussverfahren h) wird vorzugsweise in Gegenwart einer Protonen-abgebenden Säure, z.B. Polyphosphorsäure, oder einer Lewis-Säure, z.B. Bortrifluorid oder Aluminiumchlorid nach für Friedel-Crafts-Alkylierungen bekannten Reaktionsbedingungen durchgeführt.

Die Herstellung von Verbindungen der Formel I gemäss den Verfahrensvarianten i) und j) erfolgt unter den für die Verfahrensvariante a) genannten Reaktionsbedingungen.

Das Ringschlussverfahren gemäss Verfahrensvariante k) erfolgt unter den für die Verfahrensvariante g) genannten Reaktionsbedingungen.

Derivatisierungsverfahren gemäss Verfahrensvariante l) erfolgen anhand bekannter Methoden des Standes der Technik.

Das Decarboxylierungsverfahren, insbesondere einer Verbindung der Formel XII, worin n null ist, zu einer Verbindung der Formel III, worin $R_1$ Wasserstoff ist, erfolgt bei erhöhter Temperatur, insbesondere in einem hochsiedenden inerten Lösungsmittel wie Aethylenglycol oder Xylol, vorzugsweise in einem Temperaturbereich von 150 bis 250°C. Anschliessende Reduktion zur Verbindung der Formel I erfolgt analog Verfahrensvariante a).

Ein Ester der Formel XII lässt sich in die freie Carbonsäure analog bekannten Verfahren, z.B. mit einer wässrigen Lösung enthaltend ein Alkalimetallhydroxid oder -cyanid, in die freie Carbonsäure umwandeln.

Verbindungen der Formel XII oder deren Ester, z.B. deren Niederalkylester, können z.B. mit Diboran in Verbindungen der Formel I umgewandelt werden, worin $R_1$ Hydroxyniederalkyl darstellt. Solche Verbindungen können analog bekannter Methoden in weitere Derivate übergeführt werden.

Die Verbindungen der Formel I, worin $R_1$ z.B. Hydroxymethyl bedeutet, lassen sich in reaktive veresterte Derivate überführen, indem man z.B. durch Kondensation mit einem Niederalkansulfonsäurehalogenid, z.B. -chlorid, Verbindungen erhält, worin $R_1$ Niederalkylsulfonyloxymethyl bedeutet. Solche Verbindungen lassen sich wie folgt weiter derivatisieren:

a') Kondensationsreaktion mit einem Niederalkanol, Niederalkylmercaptan, Arylniederalkylmercaptan, Ammoniak, einem Mono- oder Diniederalkylamin, vorzugsweise in Gegenwart einer Base. Man erhält Verbindungen der Formel I, worin $R_1$ Niederalkoxymethyl, Niederalkylthiomethyl, Arylniederalkylthiomethyl, Aminomethyl oder Mono-oder Diniederalkylaminomethyl bedeuten.

b') Die Reduktion mit einem Reduktionsmittel wie Lithiumtriäthylborhydrid ergibt Verbindungen der Formel I, worin $R_1$ Methyl ist.

c') Die Kondensation mit einem Alkalimetallcyanid, z.B. Kaliumcyanid, ergibt Verbindungen der Formel I, worin $R_1$ Cyanomethyl ist. Diese können dann ihrerseits in Carbonsäuren der Formel XII überführt werden, worin n eins ist. Weitere Derivatisierungsreaktionen, welche weiter vorn für Carbonsäuren der Formel XII beschrieben sind, worin n Null ist, ergeben Verbindungen, worin $R_1$ z.B. Niederalkoxyäthyl, Niederalkylthioäthyl, Aryl-niederalkylthioäthyl, Aminoäthyl oder Mono- oder Diniederalkylaminoäthyl bedeuten können.

d') Die Kondensation mit einem Diniederalylmalonsäureester, z.B. Diäthylmalonat, in Gegenwart einer Base, z.B. Natriumäthanolat, Hydrolyse des erhältlichen Malosäureesters und Decarboxylierung der substituierten Malonsäure ergibt Carbonsäuren der Formel XII, worin n zwei ist. Weitere Derivatisierungsreaktionen, wie weiter vorn für Verbindungen der Formel XII beschrieben, worin n null ist, führen zu Verbindungen der Formel I, worin $R_1$ z.B. Niederalkoxypropyl, Niederalkylthiopropyl, Arylniederalkylthiopropyl, Aminopropyl, oder Mono- oder Dialkylaminopropyl bedeutet.

Erfindungsgemässe Verbindungen der Formeln I und II und deren Vorstufen können in andere Verbindungen der Formel I oder entsprechende Vorstufen analog bekannten Verfahren umgewandelt werden.

Verbindungen der Formeln I und II, worin R und R' Wasserstoff bedeutet, können in die entsprechenden Niederalkyl-, Niederalkenyl-, Niederalkinyl-, Aroylniederalkyl- oder Arylniederalkylderivate überführt werden und zwar durch folgende Verfahren:

a") Umsetzung mit einem reaktionsfähigen Esterderivat, z.B. einem Halogenid, des entsprechenden

Niederalkanols, Niederalkenols, Niederalkinols, Aroylniederalkanols oder Arylniederalkanols;

b") Die Umsetzung mit einem entsprechenden Aldehyd, z.B. Niederalkylaldehyd, Arylniederalkylaldehyd oder einem Arylaldehyd ergibt Verbindungen der Formel I, worin R oder R' Niederalkyl oder Arylniederalkyl bedeuten, wobei man in Gegenwart eines Reduktionsmittels wie Natriumcyano-borhydrid arbeitet.

c") Die reduktive Alkylierung mit Formaldehyd oder Ameisensäure ergibt Verbindungen, worin R oder R' Methyl bedeutet.

Verbindungen der Formeln I oder II worin R oder R' Methyl bedeuten, können ebenfalls durch Umsetzung der entsprechenden Wasserstoffverbindungen (R oder R' = H) mit einem Niederalkyl- oder Phenylniederalkylchlorameisensäureester umgesetzt werden, worauf man die entsprechenden Niederalkoxycarbonyl- oder Phenylniederalkoxycarbonyl-Verbindungen erhält, die seinerseits durch Umsetzung mit einfachen oder komplexen Leichtmetallhydriden, z.B. Lithiumalumin iumhydrid oder Natrium-tris-tert-butoxy- oder -bis-(2-methoxyäthoxy)-aluminiumhydrid in die Methylverbindungen umgewandelt werden.

Verbindungen der Formel I, die am Ring A durch Hydroxy substituiert sind, und zwar die entsprechenden Phenolderivate, können analog bekannten Verfahren in verätherte Hydroxyderivate übergeführt werden. So kann man die entsprechende Hydroxyverbindung in Gegenwart einer Base, z.B. unter Phasen-Transfer-Katalyse-Bedingungen, mit einem reaktionsfähigen Esterderivat des veräthernden Alkohols, z.B. dem Halogenid, umsetzen. Man kann auch den Alkohol selbst einsetzen. Dabei verwendet man ein Kondensationsmittel wie Dicyclohexylcarbodiimid.

Weitere verätherte Hydroxyderivate von Verbindung der Formel I und zwar die dimeren Derivate der Formel Ib erhält man durch Umsetzung mit Methanderivaten, die sich formal von $CH_2(OH)_2$ ableiten, z.B. mit Diiodomethan, in Gegenwart einer Base und vorzugsweise unter Phasen-Transfer-Katalyse-Bedingungen.

Halogen, insbesondere Brom oder Chlor, kann in die Ringgruppierung durch Behandlung mit elementarem Chlor oder Brom eingeführt werden, wobei man ein polares Lösungsmittel wie Essigsäure verwendet.

Verbindungen der Formel I, in denen der Ring A beispielsweise durch Acyloxy substituiert ist, wie durch $C_{1-7}$-Alkanoyloxy oder Aroyloxy, können durch Hydrolyse mit z.B. wässriger Säure, wie Salzsäure, oder mit wässrigem Alkalihydroxid, wie Lithium- oder Natriumhydroxid, in Verbindungen der Formel I übergeführt werden, in denen Ring A durch Hydroxy substituiert ist.

Umgekehrt können Verbindungen der Formel I, in denen z.B. der Ring A durch Hydroxy substituiert ist, in Verbindungen der Formel I, in denen Ring A durch Acyloxy wie $C_{1-7}$-Alkanoyloxy oder Aroyloxy substituiert ist, umgewandelt werden, indem man erstere mit einer entsprechenden Carbonsäure oder mit einem reaktiven Derivat davon auf für Acylierungen (Veresterungen) an sich bekannter Weise kondensiert.

Die Umwandlung von Verbindungen der Formeln I, in denen Ring A durch verethertes Hydroxy, wie $C_{1-7}$-Alkoxy, substituiert ist, in Verbindungen der Formel I, in denen der Ring A durch Hydroxy substituiert ist, wird auf an sich bekannte Weise durchgeführt, z.B. mit einer Mineralsäure, wie Iodwasserstoffsäure, oder, vorzugsweise für Verbindungen, in denen $C_{1-7}$-Alkoxy für Methoxy steht, mit Bortribromid in Dichlormethan oder mit Natrium- oder Lithiumdiphenylphosphid in Tetrahydrofuran.

Die Umwandlung von Verbindungen der Formel I, worin die Ringgruppierung A durch eine gegebenenfalls substituierte Benzyloxygruppierung substituiert ist, in die entsprechende Hydroxyverbindungen kann beispielsweise hydrogenolytisch mit Wasserstoff in Gegenwart eines Katalysators, z.B. Palladium, erfolgen.

Analog können Verbindungen der Formel I, worin R oder R' Benzyl oder substituiertes Benzyl bedeuten, hydrogenolytisch in die entsprechenden Wasserstoffderivate (R und R' = H) mit Wasserstoff in Gegenwart eines geeigneten Katalysators, z.B. Palladium auf Holzkohle, übergeführt werden.

Ungesättigte Verbindungen, z.B. Verbindungen der Formel I mit einem Alkenyl- oder Alkinylrest, können ebenfalls mit katalytisch aktiviertem Wasserstoff in die entsprechenden Alkylderivate der Formel I übergeführt werden.

Verbindungen der Formel I mit einem Halogensubstituenten, insbesondere Brom, am Ring A können in die entsprechenden Niederalkylderivate durch Umsetzung mit einer starken Base wie Butyllithium und Umsetzung mit einem elektrophilen Reagenz wie einem Alkylhalogenid übergeführt werden.

Ausgangsmaterialien der Formeln III und XII kann man bevorzugt durch Zyklisierung von Verbindungen der Formel

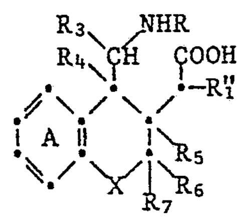

(XIII)

oder eines reaktionsfähigen, funktionellen Derivats davon und worin in obiger Formel der Ring A, X, R und $R_3$ bis $R_7$ die weiter vorn genannten Bedeutungen haben, und $R_1''$ die Bedeutungen von $R_1$ und $R_1'$ in den Formeln I und II hat oder die Gruppierung $-(CH_2)_n-COOH$ darstellt, analog bekannten Verfahren zur Herstellung einer Amidbindung herstellen.

Verbindungen der Formel XIII, worin $R_3$ Wasserstoff ist, können in situ durch Reduktion von entsprechenden Nitrilen der Formel

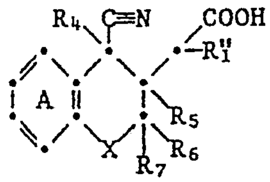

(XIV),

worin die Ringgruppierung A, X, $R_1''$ und $R_4$ - $R_7$ die angegebenen Bedeutungen haben, hergestellt werden.

So können insbesondere gegebenenfalls substituierte Chroman-4-one oder Thiochroman-4-one beispielsweise mit Trimethylsilylcyanid kondensiert werden, worauf man 4-Cyano-2H-[1]benzopyrane oder 4-Cyano-2H-[1]thiobenzopyrane erhält. Die anschliessende Kondensation dieser Verbindungen mit einem Malonsäurediniederalkylester ergibt Verbindungen der Formel XIV, worin $R_1''$ Carboxy ist, und zwar in derivatisierter Form als Diniederalkylester. Die Reduktion dieser Verbindung kann beispielsweise mit Natriumborhydrid in Gegenwart von Kobaltchlorid oder mittels katalytischer Hyerierung, z.B. mit Platin in Eisessig, erfolgen, worauf man Verbindungen der Formel XIII erhält, welche man zyklisieren kann und welche entsprechende Niederalkylester von Verbindungen der Formel XII ergeben, worin R Wasserstoff und n null ist. Diese Verbindungen können in die geometrischen cis/trans-Isomere aufgetrennt und wie unter Verfahrensvariante 1) beschrieben derivatisiert werden.

Ausgangsmaterialien der Formel XIV, worin $R_1''$ Carboxy darstellt, und worin die Substituenten am Heterocyclus in trans-Stellung sich befinden, können wie folgt hergestellt werden:

Gegebenenfalls substituierte 2H-[1]-Benzopyrane oder 2H-[1]-Benzothiopyrane werden beispeilsweise mit Dimethyldiazomalonat in Gegenwart des Kupfer(I)-jodid-Trimethylphosphit-Komplexes umgesetzt, worauf man die cis-Cyclopropyl-kondensierten Verbindungen erhält, insbesondere den Dimethylester einer cis-1,1a,2,7b-Tetrahydrobenzo[b]cyclopropa[d]pyran-1,1-dicarbonsäure. Anschliessende Oeffnung des Cyclopropylrings, insbesondere mit Diäthylaluminiumcyanid, ergibt das trans-Isomere des Dimethylesters von Verbindungen der Formel XIV, worin $R_1''$ Carboxy bedeutet.

Ausgangsmaterialien der Formeln IV oder IVa können insbesondere in situ aus Estern von 4-Carboxy-2H-[1]benzothio-pyranen hergestellt werden und zwar durch Michael-Addition mit einem alpha-($R_1$-substituierten)-Malonsäureester, Decarboxylierung und Reduktion der entsprechenden Dicarbonsäure oder des Esters davon zum entsprechenden Diol, Umwandlung in ein reaktionsfähiges Derivat, z.B. in das Dimesylat, und Kondensation mit einem Amin $RNH_2$, worin R wie oben definiert ist.

Ausgangsmaterialien der Formeln IX und IXa, worin T Oxo bedeutet, können durch Kondensation des erwähnten Dicarbonsäure-Zwischenprodukts oder als entsprechenden Esters davon mit einem Amin $RNH_2$ hergestellt werden.

Ausgangsmaterialien der Formeln V und Va können analog dem aus der U.S. Patentschrift 3,535,327 bekannten Verfahren und gegebenenfalls durch Hydrierung der Doppelbindung an der Ringverknüpfung hergestellt werden.

Ausgangsmaterialien der Formel VII können durch Reduktion einer Carboxy- oder funktionalisierten Carboxygruppe in entsprechend substituierten 3-(o-Hydroxyphenyl)-4-carboxypiperidinen wie weiter vorn beschrieben zu entsprechenden Alkoholen hergestellt werden, welche dann in reaktionsfähige, veresterte Derivate überführt werden können.

Ausgangsmaterialien der Formel VI, worin R Wasserstoff ist, können durch Kondensation mit entsprechend substituierten 4-Chlormethylpyridin-Derivaten und Phenolen zu 4-Phenoxymethylpyridinen hergestellt werden, welche dann mit einer Lewis-Säure umgesetzt werden. Quaternäre Salze erhält man durch anschliessende N-Alkylierung.

Ausgangsmaterialien für die Verfahrensvariante b), worin z.B. sich eine Doppelbindung an der Ring-Verknüpfungsstelle befindet, können durch Reduktion von Ausgangsmaterialien der Formel VI, z.B. mit Diboran oder Natriumborhydrid, erhalten werden.

Ausgangsmaterialien der Formel VIII sind z.B. durch selektive Reduktion mit einem Borhydridreduktionsmittel, z.B. Diboran oder Natriumborhydrid, einer entsprechend substituierten 4-Phenoxymethylpyridin- oder -pyridinium-Verbindung zugänglich.

Ausgangsmaterialien der Formel IX sind beispielsweise durch Michael-Addition beispielsweise eines Niederalkylesters von 4-Carboxy-2H-[1]benzopyran oder 4-Carboxy-2H-[1]benzothiopyran mit einem geeignet substituierten alpha-($R_1$-substituierten)-Cyanessigsäureester zugänglich und zwar durch Hydrolysieren und Decarboxylieren des Diesters, Reduktion der Cyanogruppe und Zyklisieren der gebildeten Aminosäure oder des Esters zu einer Verbindung der Formel IX, worin R Wasserstoff bedeutet, und, wenn nötig, N-Alkylierung von Verbindungen der Formel IX.

Ausgangsmaterialien der Formel X können durch N-Acylierung von Verbindungen der Formel I, worin R Wasserstoff bedeutet, mit einer Carbonsäure der Formel $R_8$-COOH, worin $R_8$ die für Verbindungen der Formel X gegebenen Bedeutungen hat, oder mit einem reaktionsfähigen funktionellen Derivat davon, hergestellt werden.

Ausgangsmaterialien der Formel XI sind durch Umwandlung von alphasubstituierten 3-Butensäureamiden in

die entsprechenden 3-Butenylamine und Kondensation dieser 3-Butenylamine mit entsprechend substituierten o-Hydroxyphenyloxiranen zu Verbindungen der Formel XI, worin X Sauerstoff bedeutet und V die Bedeutung von Hydroxy hat, zugänglich. Die Umwandlung in die entsprechenden Verbindungen, worin V reaktionsfähiges, verestertes Hydroxy darstellt, z.B. Halogen, kann anhand bekannter Methoden vorgenommen werden.

Die zur Gruppe der 2H-[1]-Benzopyrane. 2H-[1]-Benzothiopyrane, Chroman-4-one und Thiochroman-4-one gehörenden Ausgangsmaterialien sind bekannt oder können in an sich bekannter Weise hergestellt werden.

Im Zusammenhang mit den vorstehend erwähnten Reaktionen kann es, wie schon erwähnt, vorteilhaft sein, die möglicherweise reaktiven Substituenten, wie Amino, Carboxy oder Hydroxy, oder andere störende Substituenten, in geeigneter Weise zu schützen. Man bedient sich dabei an sich bekannter Schutzgruppen-Techniken, wie z.B. nachstehend illustriert, um Nebenreaktionen zu vermeiden, indem man solche Substituenten vor der gewünschten Reaktion schützt und danach, wenn nötig, die Schutzgruppen wieder abspaltet, um die gewünschten Verbindungen, z.B. solche der Formel I oder auch Zwischenprodukte, zu erhalten.

So kann eine freie Aminogruppe, die zumindest ein Wasserstoffatom am Stickstoff trägt, in Form eines leicht spaltbaren Amides geschützt werden, z.B. als Acylderivat, wie als Benzyloxycarbonyl- oder als tert.-Butoxycarbonyl-Verbindung, oder durch jede andere leicht abspaltbare N-Schutzgruppe.

Eine Carboxygruppe lässt sich in Form eines leicht spaltbaren Esters schützen, z.B. als Benzyl- oder tert.-Butylester, oder als Ester, der sonst üblicherweise verwendet wird.

Eine Hydroxygruppe kann ebenfalls als Ester geschützt vorliegen, z.B. als Acylderivat, wie als $C_{1-7}$-Alkanoyl-, Benzyloxycarbonyl-oder $C_{1-7}$-Alkoxycarbonylester. Ferner kann eine solche Hydroxygruppe als Ether geschützt werden, z.B. als 2-Tetrahydropyranyl- oder als Benzylether.

In einer erhaltenen Verbindung der Formel I oder einem Zwischenprodukt, in dem eine oder mehrere funktionelle Gruppen geschützt sind, werden solche geschützten funktionellen Gruppen, z.B. Amino-, Hydroxy- oder Carboxygruppen, auf an sich bekannte Weise freigesetzt, z.B. durch Solvolyse, insbesondere saure Hydrolyse, oder durch Reduktion, insbesondere Hydrogenolyse.

Die vorstehend erwähnten Umsetzungen werden nach Standardverfahren durchgeführt, in Gegenwart von oder ohne Lösungsmittel, vorzugsweise solchen, die gegenüber den Reagenzien inert sind und sie zu lösen vermögen, von Katalysatoren, Kondensationsmitteln oder anderen Stoffen und/oder unter Schutzgasatmosphäre, bei niedrigen Temperaturen, Raumtemperatur oder erhöhter Temperatur, vorzugsweise im Bereich des Siedepunktes der verwendeten Lösungsmittel, bei atmosphärischem oder erhöhtem Druck.

Die Erfindung umfasst ferner jede Varianten der erwähnten Verfahren, nach der ein Zwischenprodukt, das sich auf irgendeiner Stufe isolieren lässt, als Ausgangsstoff verwendet wird, um damit die weiteren Reaktionsschritte durchzuführen, oder nach der Ausgangs stoffe unter den Reaktionsbedingungen gebildet werden oder nach denen Ausgangsstoffe in Form ihrer Salze verwendet werden oder als optisch reine Antipoden. Immer wenn es wünschenswert ist, werden vor Ausführung der beschriebenen Verfahren möglicherweise störende reaktive funktionelle Gruppen auf geeignete Weise geschützt, wie vorstehend und in den Beispielen illustriert.

Vorzugsweise werden in diesen Umsetzungen solche Ausgangsstoffe verwendet, die zur Bildung der vorstehend als bevorzugt bezeichneten Verbindungen führen.

Die Erfindung bezieht sich auch auf neue Ausgangsstoffe und Verfahren zu deren Herstellung.

Je nach Wahl der Ausgangsstoffe und Verfahren lassen sich die neuen Verbindungen in Form eines der möglichen Isomere oder als Isomerengemisch gewinnen, z.B. als reine geometrische Isomere (cis oder trans), als reine optische Isomere (als Antipoden), oder als Mischungen optischer Isomere, wie als Racemate, oder als Mischungen geometrischer Isomere.

Verbindungen der Formel I oder II mit einer trans-4a,10b-ring-Verknüpfung können aus Verbindungen der Formel I oder II mit einer cis-4a,10b-Ring-Verknüpfung hergestellt werden, indem man sie mit einer starken Base, wie Kalium-tert.-butylat, in einem nicht wässrigen Lösungsmittel, wie Dimethylsulfoxid, behandelt, und, wenn erwünscht, die trans-verknüpfte Verbindung aus einer Mischung der Isomere, z.B. durch Chromatographie oder durch Kristallisation, abtrennt.

Erhaltene Gemische von geometrischen Isomeren oder von Diastereomeren von Verbindungen der Formel I oder II oder von Zwischenprodukten davon können auf an sich bekannte Weise in die einzelnen racemischen oder optisch aktiven Isomere aufgetrennt werden, z.B. durch fraktionierte Destillation oder Kristallisation und/oder durch Chromatographie.

Racemische Produkte der Formeln I oder II sowie basische Zwischenprodukte können z.B. durch Auftrennung von ihren diastereomeren Salzen in die optischen Antipoden aufgetrennt werden, z.B. durch fraktioniertes Kristallisieren ihrer d- oder l-(Tartrate, Dibenzoyltartrate, Mandelate oder Camphersulfonate).

Jedes Zwischenprodukt mit einer sauren Gruppe, welches zur Bildung von Salzen befähigt ist, kann z.B. durch Trennung mit optisch aktiven Basen wie 1-α-Methylbenzylamin, Cinchonidin, Cinchonin, Chinin, Chinidin, Ephedrin, Dehydroabietylamin, Brucin oder Strychnin in reiner Form isoliert werden.

Vorzugsweise wird das aktivere Isomer, wie ein Antipode, von den Verbindungen dieser Erfindung isoliert.

Die Verbindungen dieser Erfindung werden entweder als freie Verbindungen oder als Salze erhalten. Jede erhaltene Base kann in ein entsprechendes Säureadditionssalz umgewandelt werden, vorzugsweise unter Verwendung einer pharmazeutisch annehmbaren Säure oder eines Anion-Austauschpräparates. Ferner können erhaltene Salze in die entsprechenden freien Basen übergeführt werden, z.B. unter Verwendung einer

stärkeren Base, wie eines Metall- oder Ammoniumhydroxids oder irgendeines basischen Salzes, z.B. eines Alkalimetallhydroxids oder -carbonats, oder eines Kation-Austauschpräparates. Diese oder andere Salze, beispielsweise Pikrate, können auch zur Reinigung der erhaltenen Basen dienen, die Basen werden dafür in die Salze übergeführt. Wegen der engen Beziehungen zwischen den freien Verbindungen und ihren Salzen ist im Rahmen dieser Erfindung im Zusammenhang mit der Erwähnung einer Verbindung immer auch ein ihr entsprechendes Salz mit gemeint und umfasst, soweit das nach den Umständen möglich und sinnvoll ist.

Die Verbindungen, wie auch ihre Salze, können auch in Form ihrer Hydrate erhalten werden oder andere Lösungsmittel erhalten, die für ihre Kristallisation verwendet wurden.

Die vorliegende Erfindung betrifft ebenfalls die therapeutische Verwendung von Verbindungen der Formeln I und II und ihrer pharmazeutisch annehmbaren, nicht-toxischen Säureadditionssalze oder von pharmazeutischen Zusammensetzungen mit diesen Wirkstoffen als Heilmittel insbesondere als Serotonin-2 Blocker (Serotonin-Antagonisten an den Serotoninrezeptoren), zur Behandlung von Störungen aufgrunde von Serotoninblockade, insbesondere psychotropen Störungen wie Angstzustände, Depressionen oder Manie, gastrointestinalen Leiden wie Ulcus oder Herzkreislauf-Beschwerden wie Bluthochdruck.

Insbesondere betrifft die vorliegende Erfindung ein Behandlungsverfahren von psychotropen Störungen in Säugern, insbesondere Menschen, als Folge von Serotonin-2 Blockade, insbesondere Angstzuständen, indem man eine wirksame Menge einer erfindungsgemässen Verbindung der Formel I oder II oder eines pharmazeutisch annehmbaren Salzes davon als Wirkstoffe vorzugsweise in Form von pharmazeutischen Zusammensetzungen anwendet.

Die zu applizierende Dosis der Wirkstoffs ist speziesabhängig und ist ausserdem vom Körpergewicht, dem Alter, dem Zustand des Patienten und der Anwendungsform abhängig.

Die Einheitsdosis für einen Säuger von ca. 50-70 kg Körpergewicht kann zwischen 5 und 100 mg des Wirkstoffs betragen.

Die vorliegende Erfindung betrifft ebenfalls die Verwendung der erfindungsgemässen Verbindungen zur Herstellung von pharmazeutischen Präparaten, vorzugsweise pharmazeutischen Präparaten mit modulierender Wirkung auf die Serotonin-2 Rezeptoren, vor allem mit Serotonin-2 Blockade Wirkung.

Die pharmazeutischen Präparate eignen sich zur enteralen, z.B. oralen oder rektalen, transdermalen und parenteralen Verabreichung an Säugern, insbesondere Menschen, zur Behandlung von Krankheiten, welche in Folge des Serotoninantagonismus an den Serotonin-2 Rezeptoren auftreten.

Es bedarf dazu einer wirksamen Menge einer pharmakologisch aktiven Verbindung der Formeln I und II oder eines pharmazeutisch annehmbaren Salzes davon, allein oder in Kombination mit einem oder mehreren pharmazeutisch annehmbaren Hilfsstoffen.

Die pharmakologisch aktiven Verbindungen dieser Erfindung lassen sich zur Herstellung von pharmazeutischen Präparaten verwenden, die eine wirksame Menge davon zusammen mit Hilfs- oder Trägerstoffen enthalten, die zur enteralen oder parenteralen Verabreichung geeignet sind. Bevorzugt sind Tabletten und Gelatinekapseln, die die aktive Komponente gemeinsam mit a) Verdünnungsmitteln, z.B. Laktose, Dextrose, Sucrose, Mannit, Sorbit, Cellulose und oder Glycin, b) Gleitmitteln, z.B. Silica, Talk, Stearinsäure, deren Magnesium-oder Calciumsalze und/oder Polyethylenglykol, für Tabletten auch c) Bindemitteln, z.B. Magnesiumaluminiumsilikat, Stärke, Gelatine, Traganth, Methylcellulose, Natriumcarboxymethylcellulose und/oder Polyvinylpyrrolidon, wenn erwünscht d) Sprengmitteln, z.B. Stärken, Agar, Alginsäure oder Natriumalginat, oder schäumende Mischungen, und/oder e) Absorbentien, Farbstoffen, Geschmacks- und Süssstoffen enthalten. Injizierbare Präparate sind vorzugsweise wässrige isotonische Lösungen oder Suspensionen; Suppositorien werden vorteilhaft aus Fettemulsionen oder Suspensionen hergestellt. Diese Präparate können sterilisiert sein und/oder Zusatzstoffe enthalten, wie Konservierungs-, Stabilisierungs-, Netz- oder Emulgiermittel, Lösungsvermittler, Salze zur Regulierung des osmotischen Drucks und/oder Puffer. Zusätzlich können die Präparate auch andere therapeutisch wertvolle Substanzen enthalten. Besagte Präparate werden mittels konventioneller Misch-, Granulier- und Ueberzugsverfahren hergestellt. Sie enthalten etwa 0,1 bis 75 %, vorzugsweise etwa 1 bis 50 % des aktiven Bestandteils.

Geeignete Formen zur transdermalen Verabreichung umfassen eine wirksame Menge einer Verbindung der Formel I, Ia oder Ib und einen Träger. Vorteilhafte Träger umfassen absorbierbare pharmakologisch annehmbare Lösungsmittel, die den Transport durch die Haut unterstützen sollen. Insbesondere bestehen transdermale Anwendungsformen aus einem Pflaster, das aus einer undurchlässigen Oberschicht besteht, einem Reservoir, in dem sich der Wirkstoff befindet, gegebenenfalls mit Trägerstoffen, gegebenenfalls einer die Abgabegeschwindigkeit regulierende Barriere, damit der Wirkstoff an die Haut des Behandelten während einer längeren Zeit in kontrollierter und vorher bestimmbarer Weise abgegeben wird, sowie einem Mittel, um die Anwendungsform auf der Haut sicher zu halten.

Die folgenden Beispiele sollen die Erfindung illustrieren und sind nicht geeignet, die Erfindung zu beschränken, etwa auf den Umfang der Beispiele. Temperaturen werden in Celsiusgraden angegeben. Alle Verdampfungsvorgänge werden, wenn nicht ausdrücklich anderweitig beschrieben, unter vermindertem Druck, vorzugsweise zwischen 2 und 13 kPa, vorgenommen. Die Struktur der Produkte, Zwischenprodukte und Ausgangsmaterialien lässt sich analytisch, z.B. mikroanalytisch oder spektroskopisch (MS, IR, NMR) bestimmen. Die Symbole R* und S* bezeichnen die relative Konfiguration an den betreffenden achiralen Kohlenstoffatomen.

Beispiel 1:

1.1 (±)-[4R*,4aS*,10bR*)-4-Hydroxymethyl-1,3,4,4a,5,10b-hexahydro-9-methoxy-2H-[1]benzopyrano[4,3-c]pyridin

a) Zu einer gerührten Suspension von 8,3 g (±)-[4R*,4aS*,10bR*]-1,3,4,4a,5,10b-hexahydro-9-methoxy-3-oxo-2H-[1]benzopyrano[4,3-c]-pyridin-4-carbonsäuremethylester in 40 ml THF werden unter Stickstoff 175 ml 1 m Diboranlösung in THF gegeben. Man erhitzt fünf Stunden lang unter Rückfluss, kühlt ab und fügt 6 ml Wasser hinzu, gefolgt von 100 ml 6 N HCl-Lösung. Das Reaktionsgemisch wird 30 Min. unter Rückfluss erhitzt, abgekühlt, eingeengt und mit 120 ml 6 N NaOH-Lösung basisch gemacht. Die wässrige Phase wird anschliessend mit sechs 50 ml Portionen Methylenchlorid extrahiert und die organische Phase über Natriumsulfat getrocknet, filtriert und eingeengt, worauf man die Titelverbindung erhält. F.p.: 282-283° (HCl-Salz).

Bei Behandlung von rohem 1,3,4,4a,5,10b-Hexahydro-9-methoxy-3-oxo-2H-[1]benzopyrano[4, 3-c]pyridine-4-carbonsäuremethylester wird in analoger Weise diastereomeres (±)-[4S*,4aS*,10bR*]-4-Hydroxymethyl-1,3,4,4a,5,10b-hexahydro-9-methoxy-2H[1]benzopyrano-[4,3-c]pyridinhydrochlorid, F.p.: 295-296° (Zers.), erhalten.

Herstellung des Ausgangsmaterials:

b) Zu einer gerührten Lösung von 79 g 6-Methoxy-2H-[1]benzopyran (J. Org. Chem. 1973, 38, 3832) und 17,6 g Kupfer(I)-jodid-Trimethylphosphit-Komplex (Synthesis 1971, 658) in 400 ml Toluol werden bei 103° 125 g Dimethyldiazomalonat (Synthesis 1971, 658) dreissig Minuten lang gegeben. Man lässt 1,5 Stunden rühren, kühlt ab und filtriert über Celite®-Filter. Man engt das Filtrat ein und chromatographiert ("Flash") mit Essigester-Hexan-3:7-Gemisch, worauf man cis-(±)-Dimethyl-1,1a,2,7b-tetrahydro-6-methoxybenzo[b]cyclopropa[d] pyran-1,1-dicarboxylat erhält.

c) Zu 93 g der gemäss b) erhältlichen Verbindung in 1250 ml Toluol gibt man 425 ml Diäthylaluminiumcyanid (1,5 M in Toluol) unter Stickstoff. Man rührt zwei Stunden lang bei 25° und giesst das Reaktionsgemisch in 1250 ml 17,4 %-ige wässrige HCl-Lösung. Man trennt die wässrige Phase ab und extrahiert diese mit drei 100 ml Portionen Essigester und drei 200 ml Portionen Methylenchlorid. Man wäscht die vereinigten organischen Phasen mit 500 ml 2N HCl-Lösung, 500 ml gesättigter, wässriger Kochsalzlösung, trocknet über Natriumsulfat, filtriert und engt ein, worauf man trans-(±)-Dimethyl-2-(3,4-dihydro-6-methoxy-4-cyano-2H-[1]benzopyran-3-yl)-malonat erhält.

d) Zu 96 g der gemäss c) erhältlichen Verbindung in 1600 ml Eisessig gibt man 10 g $PtO_2$ hinzu. Anschliessend hydriert man zwei Stunden lang die Verbindung bei 45 psi. Man filtriert das Reaktionsgemisch über eine Celitschicht und engt das Filtrat ein. Man nimmt den Rückstand in 100 ml siedendem Methanol auf und lässt abkühlen. Man filtriert die erhaltene Verbindung ab, wäscht diese mit 50 ml kaltem Methanol-Aether-1:1-Gemisch und trocknet im Vakuum, worauf man (±)-[4R*,4aS*,10bR*]-1,3,4,4a,5,10b-Hexahydro-9-methoxy-3-oxo-2H-[1]benzopyrano-[4,3-c]pyridin-4-carbonsäuremethylester erhält. F.p.: 206-210°.

Analog lassen sich folgende Verbindungen herstellen:

1.2 (±)-[4R*,4aS*,10bR*]-4-Hydroxymethyl-1,3,4,4a,5,10b-hexahydro-7-methoxy-2H-[1]benzopyrano-[4,3-c]pyridin, F.p.: 135-145° (HCl-Salz);

1.3 (±)-[4R*,4aS*,10bR*]-4-Hydroxymethyl-1,3,4,4a,5,10b-hexahydro-10-methoxy-2H-[1]benzopyrano-[4,3-c]pyridin, F.p.: 233-234° (HCl-Salz).

Beispiel 2:

2.1 <u>(±)-[4R*,4aS*,10bR*]-7-Brom-4-hydroxymethyl-1,3,4,4a,5,10b-hexahydro-9-methoxy-2H-[1]benzopyrano[4,3-c]pyridin</u>

Zu einer gerührten Lösung von 8,66 g (±)-[4R*,4aS*,10bR*]-4-Hydroxymethyl-1,3,4,4a,5,10b-hexahydro-9-methoxy-2H-[1]benzopyrano[4,3-c]-pyridin in 90 ml Essigsäure werden 1,96 ml Brom bei 25° gegeben. Man rührt 1,5 Stunden lang, engt die Lösung ein und behandelt den Rückstand mit 100 ml 2N NaOH-Lösung. Man extrahiert mit Methylenchlorid, trocknet die organischen Phasen über Natriumsulfat, filtriert, engt ein und chromatographiert den Rückstand ("Flash") mit einem Methylenchlorid-Methanol (Ammoniak-gesättigt)-90:10-Gemisch, worauf man die Titelverbindung als Hauptprodukt erhält. F.p.: 281-285° (HCl-Salz, Zers.).

Als Nebenpodukte erhält man:

2.2 (±)-[4R*,4aS*,10bR*]-7,10-dibrom-4-hydroxymethyl-1,3,4,4a,5,10b-hexahydro-9-methoxy-2H-[1]benzopyrano[4,3-c]pyridin, F.p.: 223-224° (freie Base);

(±)-[4R*,4aS*,10bR*]-8-Brom-4-hydroxymethyl-1,3,4,4a,5,10b-hexahydro-9-methoxy-2H-[1]benzopyrano-[4,3-c]pyridin, F.p.: 268-270° (HCl-Salz, Zers.).

Beispiel 3:

(±)-[4R*,4aS*,10bR*]-7-Brom-4-hydroxymethyl-1,3,4,4a,5,10b-hexahydro-9-methoxy-2-methyl-2H-[1]benzopyrano[4,3-c]pyridin

Eine Lösung von 1,65 g (±)-[4R*,4aS*,10bR*]-7-Brom-4-hydroxymethyl1,3,4,4a,5,10b-hexahydro-9-methoxy-2H-[1]benzopyrano[4,3-c]pyridin in 37 %-iger wässriger Formaldehyd-Lösung und 2,2 ml Ameisensäure wird bei 100° 20 Minuten lang erhitzt. Man engt das Reaktionsgemisch ein und separiert dieses zwischen 10 ml 2N Natronlauge und 50 ml Methylenchlorid. Man trocknet die organische Phase über Natriumsulfat, filtriert und engt ein, worauf man die Titelverbindung erhält. F.p. des HCl-Salzes: 262° (Zers.).

Beispiel 4:

(±)-[4R*,4aS*,10bR*]-7-Brom-4-äthylthiomethyl-1,3,4,4a,5,10b-hexahydro-9-methoxy-2-methyl-2H-[1]benzopyrano[4,3-c]pyridin

a) Zu einer gerührten Lösung von 46 ml Aethylmercaptan in 40 ml trockenem THF werden unter Stickstoff bei -10° tropfenweise 2,3 ml 2,28 molare n-Butyllithium-Lösung in Hexan gegeben. Man gibt eine Lösung von 1,91 g (±)-[4R*,4aS*,10bR*]-7-Brom-4-methylsulfonyloxymethyl-1,3,4,4a,5,10b-hexahydro-9-methoxy-2-methyl-2H-[1]benzopyrano[4,3-c]pyridin in 25 ml THF hinzu. Man rührt zwei Stunden lang bei 25°, fügt 5 ml Wasser hinzu und engt das Reaktionsgemisch ein. Der Rückstand wird zwischen 25 ml Wasser und 50 ml Methylenchlorid separiert. Die organische Phase wird mit Wasser gewaschen, über Natriumsulfat getrocknet, filtriert und eingeengt. Man chromatographiert den Rückstand ("Flash") mit einem Methylenchlorid-Methanol-40:1-Gemisch und erhält ein Oel, das man in 40 ml Isopropanol löst. Man erwärmt die Lösung auf 50° und fügt ein Gemisch aus 3,4 N HCl-Lösug in Wasser und Essigester hinzu. Nach Abkühlen filtriert man das gebildete Produkt, wäscht dieses mit einem Isopropanol-Aether-1:1-Gemisch und trocknet die gereinigte Titelverbindung. F. p.: 268-269° (HCl-Salz, Zers.).

Herstellung des Ausgangsmaterials:

b) Zu einer Lösung von 1,69 g (±)-[4R*,4aS*,10bR*]-7-Brom-4-hydroxymethyl-1,3,4,4a,5,10b-hexahydro-9-methoxy-2-methyl-2H-[1]benzopyrano[4,3-c]pyridin und 0,82 ml Triäthylamin in 60 ml Methylenchlorid werden 0,42 ml Mesylchlorid gegeben. Nach 15 Minuten Reaktionszeit fügt man einen Tropfen Methanol hinzu, wäscht das Reaktionsgemisch mit gesättigter wässriger Natriumhydrogencarbonat- Lösung und trocknet die organischen Phasen über Natriumsulfat. Nach Filtration und Einengen erhält man das in 4a) verwendete Ausgangsmaterial.

Beispiel 5:

(±)-[4aR*,10bS*]-1,3,4,4a,5,10b-Hexahydro-2H-[1]benzopyrano [4,3-c]-pyridin

a) Zu einer Suspension von 0,181 g Lithiumalanat in 40 ml THF wird eine Lösung von 0,59 g (±)-[4aR*,10bS*]-1,3,4,4a,5,10b-Hexahydro-3-oxo-2H-[1]benzopyrano[4,3-c]pyridin in 5 ml THF gegeben. Man lässt drei Stunden lang bei 50° rühren, kühlt das Reaktionsgemisch ab und setzt 0,18 ml Wasser hinzu, gefolgt von 0,18 ml 15%-iger Natronlauge und 0,54 ml Wasser. Das Gemisch wird über Natriumsulfat getrocknet, über Celit® filtriert und eingedampf, worauf man die Titelverbindung erhält. F.p.: 314-318° (HCl-Salz, Zers.).

Herstellung des Ausgangsmaterials:

b) 11 g 2H-[1]-Benzopyran-4-on werden in 33 ml einer 2,5 molaren Trimethylsilylcyanid-Lösung in Methylenchlorid gelöst. Man fügt 50 mg $ZnJ_2$ hinzu und rührt die Mischung 12 Stunden lang. Man fügt 30 ml Toluol, 115 ml Pyridin und 21 ml Phosphoroxychlorid hinzu und erhitzt die Mischung vier Stunden lang unter Rückfluss. Man lässt abkühlen und giesst das Gemisch in 100 ml 5%-ige HCl-Lösung und extrahiert mit Aether. Die organische Phase wird über Natriumsulfat getrocknet, filtriert, eingeengt und Vakuum-destilliert, worauf man 4-Cyano-2H[1]benzopyran erhält.

c) 7,45 g 4-Cyano-2H-[1]benzopyran und 5,5 ml Malonsäuredimethylester in 15 ml Methanol werden bei 25° zu einer Lösung von 1,3 g Natrium in 50 ml Methanol gegeben. Man lässt dreissig Minuten stehen, fügt 30 ml Essigsäure hinzu und engt das Reaktionsgemisch ein. Man separiert das Reaktionsgemisch zwischen Wasser und Essig ester (3:1), trocknet die organische Phase, filtriert und engt ein, worauf man ein 1:1 Gemisch von cis- und trans-(±)-2-(3,4-dihydro-4-cyano-2H-[1]benzopyran-3-yl)-malonsäuredimethylester erhält.

d) Zu einer Lösung von 13,7 g der gemäss c) erhältlichen Verbindung in 350 ml Methanol, der man 30 g gemahlenes $Co(OAc)_2$ zugesetzt hat, fügt man in einem Zeitraum von 30 Minuten 13 Natriumborhydrid in mehreren Portionen hinzu. Das Reaktionsgemisch wird bis auf 50 ml Volumen eingeent und zwischen 500 ml Essigester und 200 ml 2 N HCl separiert. Man wäscht die organische Phase mit konzentrierter, wässriger Kochsalzlösung, trocknet über Natriumsulfat, filtriert und engt ein. Der Rückstand wird mit einem Aether-Essigester-Gemisch verrieben, worauf man (±)-[4R*,4aS*,10bR*]-1,3,4,4a,5,10b-Hexahydro-3-oxo-2H-[1]benzopyrano[4,3-c]pyridin-4-carbonsäuremethylester erhält.

e) Zu einer Lösung von 0,195 g KCN in 25 ml Wasser werden 0,41 g der gemäss d) erhältlichen Verbindung gegeben. Man erhitzt unter Rückfluss und fügt anschliessend 1,6 ml 2 N HCl-Lösung hinzu. Man engt ein und erhitzt den Rückstand 10 Minuten lang. Der gebildete Feststoff wird mit 60 ml heissem Aethanol extrahiert. Nach dem Eindampfen auf 15 ml erhält man nach Abkühlen und Filtrieren (±)-[4aR*,10bS*]-1,3,4,4a,5,10b-Hexahydro-3-oxo-2H-[1]-benzopyrano[4,3-c]pyridin.

Beispiel 6:

(±)-[4aR*,10bS*]-1,3,4,4a,5,10b-Hexahydro-2-propyl-2H-[1]benzopyrano[4,3-c]pyridin

Eine Lösung von 0,235 g (±)-[4aR*,10bS*]-1,3,4,4a,5,10b-Hexahydro-2H-[1]benzopyrano[4,3-c]pyridin in 5 ml Toluol wird mit 200 mg Propyljodid und 3 ml wässriger Natriumhydrogencarbonat-Lösung zwei Stunden lang erhitzt. Man trennt die organische Phase ab, trocknet über Natriumsulfat, filtriert, engt ein und chromatographiert ("Flash") den Rückstand mit einem Methylenchlorid-Methanol(Ammoniak-gesättigt)-95:5-Gemisch und erhält die Titelverbindung. F.p.: 245-247° (HCl-Salz).

Beispiel 7:

(±)-[4aR*,10bS*]-1,3,4,4a,5,10b-Hexahydro-10-hydroxy-2-propyl-2H-[1]benzopyrano[4,3-c]pyridin

Zu einer Lösung von 0,48 g Diphenylphosphin in 4 ml THF wird bei 0° unter Stickstoff 1,38 ml 2,24 molares n-Butyllithium in Hexan hinzugefügt. Nach drei Minuten Reaktionszeit gibt man 0,238 g (±)-[4aR*,10bS*]-1,3,4,4a,5,10b-Hexahydro-1-methoxy-2-propyl-2H-[1]benzopyrano[4,3-c]pyridin in 10 ml THF hinzu. Man erhitzt die Mischung 1,5 Stunden unter Rückfluss und kühlt ab. Man gibt 8 ml 2 N HCl-Lösung hinzu und dampft ein. Man extrahiert den Rückstand mit Aether, macht mit Natriumhydrogencarbonat basisch und extrahiert mit Methylenchlorid. Die organischen Phasen werden über Natriumsulfat getrocknet, filtriert und eingedampft. Man chromatographiert den Rückstand mit einem Methylenchlorid-Methanol (Ammoniak-gesättigt)-20:1-Gemisch und erhält die Titelverbindung. F.p.: 264-265° (HCl-Salz, Zers.).

Beispiel 8:

Analog den weiter vorn in den Beispielen beschriebenen Verfahren lassen sich folgende Verbindungen herstellen:

8.1 (±)-[4R*,4aS*,10bR*]-7-Brom-4-methylthiomethyl-1,3,4,4a,5,10b-hexahydro-9-methoxy-2-methyl-2H-[1]benzopyrano[4,3-c]pyridin-hydrochlorid, F.p.: 279-280° (Zers.);

8.2 (±)-[4R*,4aS*,10bR*]-7-Brom-4-[n-propylthiomethyl)-1,3,4,4a,5,10b-hexahydro-9-methoxy-2-methyl-2H-[1]benzopyrano[4,3-c]pyridin-hydrochlorid, F.p.: 252-25° (Zers.);

8.3 (±)-[4R*,4aS*,10bR*]-7-Brom-4-(n-butylthiomethyl)1,3,4,4a,5,10b-hexahydro-9-methoxy-2-methyl-2H-[1]benzopyrano[4,3-c]pyridin-hydrochlorid, F.p.: 235-236° (Zers.);

8.4 (±)-[4R*,4aS*,10bR*]-7-Brom-4-methoxymethyl-1,3,4,4a,5,10b-hexahydro-9-methoxy-2-methyl-2H-[1]benzopyrano[4,3-c]pyridinhydrochlorid, F.p.: 281-282° (Zers.);
Man verwendet Kaliummethylat statt Aethylmercaptan/Butyllithium wie im Beispiel 4a);

8.5 (±)-[4R*,4aS*,10bR*]-7-Brom-4-cyanomethyl-1,3,4,4a,5,10b-hexahydro-9-methoxy-2-methyl-2H-[1]benzopyrano[4,3-c]pyridin-hydrochlorid, F.p.: 315° (Zers.);
Man verwendet Kaliumcyanid statt Aethylmercaptan/Butyllithium wie im Beispiel 4a);

8.6 (±)-[4R*,4aS*,10bR*]-7-Brom-4-phthalimidomethyl-1,3,4,4a,5,10b-hexahydro-9-methoxy-2-methyl-2H-[1]benzopyrano[4,3-c]pyridin-hydrochlorid, F.p.: 273-275° (Zers.);
Man verwendet Kaliumphthalimid statt Aethylmercaptan/Butyllithium wie im Beispiel 4a);

8.7 (±)-[4R*,4aS*,10bR*]-4-Methylthiomethyl-1,3,4,4a,5,10b-hexahydro-2-propyl-2H-[1]benzopyrano[4,3-c]pyridin-hydrochlorid, F.p. 220-222°;

8.8 (±)-[4aR*,10bS*]-1,3,4,4a,5,10b-Hexahydro-10-methoxy-2-propyl-2H-[1]benzopyrano[4,3-c]pyridin-hydrochlorid, F.p. 252°;

8.9 (±)-[4aR*,10bS*]-7-Brom-1,3,4,4a,5,10b-hexahydro-9-methoxy-2-methyl-2H-[1]benzopyrano[4,3-c]pyridin-hydrochlorid, F.p. 307°.

Beispiel 9:

(±)-[4aR*,10bS*]-7-Brom-1,3,4,4a,5,10b-hexahydro-9-hydroxy-2-methyl-2H-[1]benzopyrano[4,3-c]pyridin

Eine Mischung von 0,5 g (±)-[4aR*,10bS*]-7-Brom-1,3,4,4a,5,10b-hexahydro-9-methoxy-2-methyl-2H-[1]benzopyrano[3,4-c]pyridin und 5 g Pyridin-hydrochlorid wird unter Stickstoff bei 200° 20 Minuten lang erhitzt. Das abgekühlte Reaktionsgemisch löst man in Wasser und macht mit 6 N NaOH-Lösung basisch. Man extrahiert die wässrige Phase mit Methylenchlorid und trocknet die organische Phase mit Natriumsulfat, filtriert, engt ein und chromatographiert den Rückstand mit einem Methylenchlorid-Methanol (Ammoniak-gesättigt)-10:1-Gemisch, worauf man die Titelverbindung erhält. F.p.: 259-262° (HCl-Salz).

Beispiel 10:

10.1
(±)-[4aR*,4aS*,10bS*]-7-Brom-1,3,4,4a,5,10b-hexahydro-9-benzyloxy-2-methyl-2H-[1]benzopyrano[4,3-c]pyridin

Zu einer Lösung von 0,380 g (±)-[4aR*10bS*]-7-Brom-1,3,4,4a,5,10b-hexahydro-9-hydroxy-2-methyl-2H-[1]benzopyrano-[4,3-c]pyridin in 6 ml Chlorbenzol werden 0,91 ml Benzylchlorid, 0,290 g Benzyltriäthylam-

moniumchlorid und 2 ml 50%-ige Natriumhydroxid-Lösung hinzugefügt. Man rührt das Reaktionsgemisch intensiv zwei Stunden lang. Man trennt die organische Phase ab, trocknet, über Natriumsulfat, filtriert und engt unter reduziertem Druck ein. Chromatographieren ("Flash") des Rückstands mit Methylenchlorid-Methanol (Ammoniak-gesättigt)-20:1-Gemisch ergibt die Titelverbindung. F.p.: 283° (HCl-Salz, Zers.).

Analog lassen sich folgende Verbindungen herstellen:

10.2 (±)-[4aR*,10bS*]-1,3,4,4a,5,10b-Hexahydro-9-(1-naphthylmethoxy)-2-methyl-2H[1]-benzopyrano[4.3-c]pyridin-hydrochlorid; F.p.:196°;

10.3 (±)-[4aR*,10bS*]-1,3,4,4a,5,10b-Hexahydro-9-(1-fluorenylmethoxy)-2-ethyl-2H[1]-benzopyrano[4,3-c]-pyridin-fumarat, F.p.:202-204°;

10.4 (±)-[4aR*,10bS*]-1,3,4,4a,5,10b-Hexahydro-9-(9-fluorenyloxy)-2-äthyl-2H[1]-benzopyrano[4,3-c]-pyridin-fumarat, F.p.: 200-202°;

10.5 Bis-[(±)-[4aR*,10bS*]-1,3,4,4a,5,10b-hexahydro-2.7-dimethyl-2H[1]-benzopyrano[4,3-c]pyridin-9-yloxy]-methan-dihydrochlorid, F.p.: 218-221°, mit Diiodomethan;

10.6 Bis-[(±)-[4aR*,10bS*]-1,3,4,4a,5,10b-hexahydro-2H[1]-benzopyrano[4,3-c]pyridin-9-yloxy]-methan-dihydrochlorid, F.p. 258° (Zers.), mit Dijodomethan;

10.4 Bis-[(±)-[4aR*,10bS*]-1,3,4,4a,5,10b-hexahydro-7-brom-2-methyl-2H[1]-benzopyrano[4,3-c]pyridin-9-yloxy]-methan-dihydrochlorid, F.p. 274-276°, mit Dijodomethan;

10.8 (±)-[4aR*,10bS*]-1,3,4,4a,5,10b-Hexahydro-7-brom-2-methyl-9-isopropyloxy-2H[1]-benzopyrano[4,3-c]-pyridin-hydrochlorid, F.p. 210-215° (Zers.).

Beispiel 11:

11.1

(±)-[4aR*,10bS*]-1,3,4,4a,5,10b-Hexahydro-9-(2-phenyläthoxy)-2-methyl-2H-[1]benzopyrano[4,3-c]pyridin

Eine Mischung von 0,2 g (±)-[4aR*,10bS*]-1,3,4,4a,5,10b-Hexahydro-9-hydroxy-2-methyl-2H-[1]benzopyrano[4,3-c]pyridin (F.p. des HCl-Salzes 268-269°), 0,215 ml 2-Phenäthylalkohol und 0,3 g Dicyclohexylcarbodiimid wird unter Stickstoff 18 Stunden lang bei 130° erhitzt. Das abgekühlte Reaktionsgemisch wird über ein Methylenchlorid-Methanol-20:1-Gemisch "Flash"-chromatographiert. F.p. der Titelverbindung: 204° (HCl-Salz).

Analog lassen sich folgende Verbindungen herstellen:

11.2 (±)-[4aR*,10bS*]-1,3,4,4a,5,10b-Hexahydro-9-[2-(l-naphthyl)-äthoxy]-2H[1]-benzopyrano[4,3-c]pyridin-hydrochlorid, F.p.: 120-125° (Zers.);

11.3 (±)-[4aR*,10bS*]-1,3,4,4a,5,10b-Hexahydro-9-[2-(2-naphthyl)-äthoxy]-2-methyl-2H[1]-benzopyrano[4,3-c]pyridin-hydrochlorid, F.p.: 136-140° (Zers.);

Beispiel 12:

(±)-[4aR*,10bS*]-1,3,4,4a,5,10b-Hexahydro-9-methoxy-2,7-dimethyl2H-[1]benzopyrano[4,3-c]pyridin

Zu einer Lösung mit 1,2 g (±)-[4aR*,10bS*]-7-Brom-1,3,4,4a,5,10b-hexahydro-9-methoxy-2-methyl-2H-[1]benzopyrano[4,3-c]pyridin in 25 ml trockenen THF werden 2,14 ml 1,98 molare n-Butyllithium-Lösung in Hexan gegeben. Nach 30 Minuten Reaktionszeit werden 36 ml Methyljodid hinzugefügt und das Reaktionsgemisch bei 25° eine Stunde lang gerührt. Man gibt 50 ml Wasser und 100 ml Essigsäureäthylester hinzu, wäscht die organische Phase mit konzentrierter, wässriger Kochsalzlösung, trocknet über Natriumsulfat, engt ein und chromatographiert den Rückstand mit einem Methylenchlorid-Methanol (Ammoniak-gesättigt)-20:1-Gemisch. Die Titelverbindung hat einen Schmelzpunkt von 263° (HCl-Salz, Zers.).

Beispiel 13:

(±)-[4R*,4aS*,10bR*]-4-Aminomethyl-7-brom-1,3,4,4a,5,10b-hexahydro-9-methoxy-2-methyl-2H[1]-benzopyrano[4,3-c]pyridin

Zu einer Suspension von 0,305 g (±)-[4R*,4aS*,10bR*]-7-Brom-1,3,4,4a,5,10b-hexahydro-9-methoxy-2-methyl-4-phthalimidomethyl-2H-[1]benzopyrano[4,3-c]pyridin in 40 ml Methanol werden 0,102 ml Hydrazinhydrat hinzugefügt. Man erhitzt die Mischung 12 Stunden lang unter Rückfluss, kühlt ab, säuert auf pH 1 mit 12 N HCl-Lösung an und konzentriert auf 3 ml Gesamtvolumen. Man verteilt den Rückstand zwischen 10 ml NaOH-Lösung und 40 ml Methylenchlorid. Die organische Phase wird über Natriumsulfat getrocknet, filtriert und eingeengt, worauf man die Titelverbindung mit einem Schmelzpunkt von 277-279° (Zers.) erhält.

Beispiel 14:

(±)-[4R*,4aS*,10bR*]-4-Benzyloxycarbonylaminomethyl-7-brom-1,3,4,4a,5,10b-hexahydro-9-methoxy-2-methyl-2H[1]-benzopyrano-[4,3-c]pyridin

Zu einer gerührten Mischung mit 0,130 g (±)-[4R*,4aS*,10bR*]-7-brom-4-aminomethyl-1,3,4,4a,5,10b-hexahydro-9-methoxy-2-methyl-2H-[1]benzopyrano[4,3-c]pyridin in 2 ml Toluol und 0,106 g $Na_2CO_3$ in 0,4 ml Wasser werden 0,06 ml Benzylchloroformat gegeben. Man rührt fünf Minuten lang, trennt die organische Phase ab trocknet über Natriumsulfat, filtriert und dampft ein, worauf man die Titelverbindung erhält; F.p.:

159-161° (HCl-Salz).

Beispiel 15:

Analog den obigen Beispielen können folgende Verbindungen hergestellt werden:

15.1 (±)-[4aR*,10bS*]-1,3,4,4a,5,10b-Hexahydro-2-methyl-2H[1]-benzopyrano[4,3-c]pyridin-hydrochlorid, F.p.: 293-300°;

15.12 (±)-[4abS*]-1,3,4,4a,5,10b-Hexahydro-8-brom-2-methyl-2H[1]-benzopyrano[4,3-c]pyridin-hydrochlorid, F.p.: 282-286°;

15.3 (±)-[4aR*,10bS*]-1,3,4,4a,5,10b-Hexahydro-7-methoxy-2H[1]-benzopyrano[4,3-c]pyridin-hydrochlorid, F.p.: 284-285°;

15.4 (±)-[4aR*,10bS*]-1,3,4,4a,5,10b-Hexahydro-7-brom-10-methoxy-2H[1]benzopyrano[4,3-c]pyridin-hydrochlorid, F.p.: 272-274°, (Zers.);

15.5 (±)-[4aR*,10bS*]-1,3,4,4a,5,10b-Hexahydro-7-brom-10-methoxy-2-methyl-2H[1]benzopyrano[4,3-c]pyridin-hydrochlorid, F.p.: 249-251°;

15.6 (±)-[4aR*,10bS*]-1,3,4,4a,5,10b-Hexahydro-10-methoxy-2-methyl-2H[1]benzopyrano[4,3-c]pyridin-hydrochlorid, F.p.: 221-222°;

15.7 (±)-[4aR*,10bS*]-1,3,4,4a,5,10b-Hexahydro-10-methoxy-2,7-dimethyl-2H[1]-benzopyrano[4,3-c]pyridin-hydrochlorid, F.p.: 212-214°;

15.8 (±)-[4aR*,10bS*]-1,3,4,4a,5,10b-Hexahydro-9-brom-10-methoxy-2,7-dimethyl-2H[1]-benzopyrano[4,3-c]pyridin-hydrochlorid, F.p.: 182-183° (Zers.);

15.9 (±)-[4aR*,10bS*]-1,3,4,4a,5,10b-Hexahydro-7-brom-10-methoxy-2-propyl-2H[1]-benzopyrano[4,3-c]pyridin-hydrochlorid, F.p.: 247-248° (Zers.);

15.10 (±)-[4aR*,10bS*]-1,3,4,4a,5,10b-Hexahydro-10-methoxy-2,7-dipropyl-2H[1]-benzopyrano[4,3-c]pyridin-hydrochlorid, F.p.: 140-141° (Zers.);

15.11 (±)-[4aR*,10bS*]-1,3,4,4a,5,10b-Hexahydro-10-methoxy-7-methyl-2-propyl-2H[1]-benzopyrano[4,3-c]pyridin-hydrochlorid, F.p.: 220-221° (Zers.);

15.12 (±)-[4aR*10bS*]-1,3,4,4a,5,10b-Hexahydro-7-brom-9-methoxy-2H[1]-benzopyrano[4,3-c]pyridin-hydrochlorid, F.p.: 302-303° (Zers.);

15.13 (±)-[4aR*,10bS*]-1,3,4,4a,5,10b-Hexahydro-9-methoxy-2-methyl-2H[1]benzopyrano[4,3-c]pyridin-hydrochlorid, F.p.: 269-270° (Zers.);

15.14 (±)-[4aR*,10bS*]-1,3,4,4a,5,10b-Hexahydro-8-brom-9-methoxy-2-methyl-2H[1]benzopyrano[4,3-c]pyridin-hydrochlorid, F.p.: 282-283° (Zers.);

15.15 (±)-[4aR*,10bS*]-1,3,4,4a,5,10b-Hexahydro-8-brom-9-hydroxy-2H[1]benzopyrano[4,3-c]pyridin-hydrochlorid, F.p.: 302° (Zers.);

15.16 (±)-[4aR*,10bS*]-1,3,4,4a,5,10b-Hexahydro-8-brom-9-methoxy-2H[1]benzopyrano[4,3-c]pyridin-hydrochlorid, F.p.: 314° (Zers.);

15.17 (±)-[4aR*,10bS*]-1,3,4,4a,5,10b-Hexahydro-7-brom-9-methoxy-2-propyl-2H[1]benzopyrano[4,3-c]pyridin-hydrochlorid, F.p.: 288° (Zers.);

15.18 (±)-[4aR*,10bS*]-1,3,4,4a,5,10b-Hexahydro-7,9-dimethoxy-2H(1)benzopyrano[4,3-c]pyridin-hydrochlorid, F.p.: 231-233° (Zers.);

15.19 (±)-[4aR*,10bS*]-1,3,4,4a,5,10b-Hexahydro-7-brom-9-methoxy-2-[3-(p-fluorobenzoyl)-propyl]-2H[1]benzopyrano[4,3-c]pyridinhydrochlorid, F.p.: 245-250° (Zers.);

15.20 (±)-[4aR*,10bS*]-1,3,4,4a,5,10b-Hexahydro-7-brom-2-äthyl-9-methoxy-2H[1]benzopyrano[4,3-c]pyridin-hydrochlorid, F.p.: 293° (Zers.);

15.21 (±)-[4aR*,10bS*]-1,3,4,4a,5,10b-Hexahydro-7-äthyl-9-methoxy-2-methyl-2H[1]benzopyrano[4,3-c]pyridin-hydrochlorid, F.p.: 236-239° (Zers.);

15.22 (±)-[4aR*,10bS*]-1,3,4,4a,5,10b-Hexahydro-9-methoxy-7-propyl-2-methyl-2H[1]benzopyrano[4,3-c]pyridin-hydrochlorid, F.p.: 188-190°;

15.23 (±)-[4aR*,10bS*]-1,3,4,4a,5,10b-Hexahydro-7-brom-9-methoxy-2-allyl-2H[1]benzopyrano[4,3-c]pyridin-hydrochlorid, F.p.: 273-274° (Zers.);

15.24 (±)-[4R*,4aS*,10bR*]-1,3,4,4a,5,10b-Hexahydro-4-äthylthiomethyl-9-methoxy-2-methyl-2H[1]benzopyrano[4,3-c]pyridin-hydrochlorid, F.p.: 192-194° (Zers.);

15.25 (±)-[4aR*,10bS*]-1,3,4,4a,5,10b-Hexahydro-7-methoxy-2-methyl-2H[1]-benzopyrano[4,3-c]pyridin-hydrochlorid, F.p.: 238° (Zers.);

15.26 (±)-[4aR*,10bS*]-1,3,4,4a,5,10b-Hexahydro-9-hydroxy-2-propyl-2H[1]-benzopyrano[4,3-c]pyridin-hydrochlorid, F.p.: 296-297° (Zers.);

15.27 (±)-[4R*,4aS*,10bR*]-1,3,4,4a,5,10b-Hexahydro-8-brom-9-methoxy-4-hydroxymethyl-2H[1]-benzopyrano[4,3-c]pyridin-hydrochlorid, F.p.: 268-270°;

15.28 (±)-[4aR*,10bS*]-1,3,4,4a,5,10b-Hexahydro-8-brom-2-methyl-2H[1]-benzopyrano[4,3-c]pyridin-hydrochlorid, F.p.: 282-286°;

15.29 (±)-[4aR*,10bS*]-1,3,4,4a,5,10b-Hexahydro-9-[2-(2-naphthyl)-äthoxy]-2H[1]-benzopyrano[4,3-c]pyridin-hydrochlorid, F.p.: 282-286°;

15.30 (±)-[4aR*,10bS*]-1,3,4,4a,5,10b-Hexahydro-9-[2-(3-n-butylphenyl)-äthoxy]-2H[1]-benzopyrano[4,3-c]pyridin-hydrochlorid, F.p.: 186-187°;

15.31 (±)-[4S*,4aS *;10bR*]-1,3,4,4a,5,10b-Hexahydro-2-methyl-9-methoxy-4-hydroxymethyl-2H[1]benzopyrano[4,3-c]pyridin-hydrochlorid, F.p.: 307° (Zers.).

Beispiel 16:

Nach den in den obigen Beispielen illustrierten Herstellungsverfahren für Verbindungen der Formel I, worin X Sauerstoff bedeutet, lassen sich Verbindungen der Formel I herstellen, worin X Schwefel bedeutet, indem man von den entsprechend substituierten 2H-[1]-Benzothiopyran-4-onen (Thiochroman-4-onen) ausgeht:

16.1 (±)-[4aR*,10bS*]-1,3,4,4a,5,10b-Hexahydro-2-methyl-2H[1]benzothiopyrano[4,3-c]pyridin-hydrochlorid, F.p.: 237-251° (Zers.);

16.2 (±)-[4aR*,10bS*]-1,3,4,4a,5,10b-Hexahydro-7-methyl-9-methoxy-2H[1]benzothiopyrano[4,3-c]pyridin-hydrochlorid, F.p.: 245-247° (Zers.);

16.3 (±)-[4aR*,10bS*]-1,3,4,4a,5,10b-Hexahydro-2,7-dimethyl-9-methoxy-2H[1]benzothiopyrano[4,3-c]pyridin-hydrochlorid, F.p.: 243-245°;

16.4 (±)-[4R*,4aS*,10bR*]-7-Brom-4-äthylthiomethyl-1,3,4,4a,5,10b-hexahydro-9-methoxy-2-methyl-2H[1]benzothiopyrano[4,3-c]pyridin;

16.5 (±)-[4aR*,10bS*]-1,3,4,4a,5,10b-Hexahydro-2H-[1]benzothiopyrano[4,3-c]pyridin-hydrochlorid, F.p.: 252-260° (Zers.);

Die als Ausgangsmaterial verwendbaren 2H-[1]Benzothiopyran-4-one lassen sich wie folgt herstellen:

a) Zu 71 g Chlor in 1000 ml Essigsäure werden bei 0° 98 g KSCN gegeben. Nach 30 Minuten Reaktionszeit fügt man 75 g m-Bromanisol hinzu und rührt die Mischung zwölf Stunden lang. Das Reaktionsgemisch wird in 4 l Wasser gegeben und mit Hexan-Aether-1:1-Gemisch extrahiert. Die vereinigten Extrakte werden mit gesättigter-wässriger Natriumhydrogencarbonat-Lösung, und mit gesättigter wässriger Kochsalzlösung gewaschen, über Natriumsulfat getrocknet, eingeengt und destilliert, worauf man 2-Brom-4-methoxyphenylthiocyanat erhält. K.p.: 140-145° (0,1 mm).

b) Zu einer Lösung von 10,8 g Natrium in 1200 ml wasserfreiem Aethanol werden unter Stickstoff 21 g der gemäss a) erhältlichen Verbindung, gelöst in 25 ml Aethanol, gegeben. Man rührt eine Stunde lang und gibt 29 ml Essigsäure hinzu. Nach Zugabe von 600 ml Wasser wird das Reaktionsgemisch mit Hexan-Aether-1:1-Gemisch extrahiert. Man wäscht die vereinigten Extrakte mit Wasser, trocknet über Natriumsulfat, filtriert und engt ein, worauf man 4-Methoxy-2-methylphenylmercaptan erhält.

c) Eine Mischung von 16,1 g 4-Methoxy-2-methylphenylmercaptan, 20 g 3-Chlorpropionsäure und 31,5 g Cäsiumfluorid in 200 ml Acetonitril wird 40 Stunden unter Rückfluss erwärmt. Man lässt abkühlen, giesst anschliessend das Reaktionsgemisch in 200 ml 1 N Natronlauge und wäscht mit Aether. Die wässrige Phase wird mit 12 N HCl-Lösung angesäuert und mit Methylenchlorid extrahiert. Man wäscht die vereinigten Extrakte mit konzentrierter, wässriger Kochsalzlösung, trocknet über Natriumsulfat, filtriert und engt ein, worauf man 3-(2-Methyl-4-methoxyphenylthio)-propionsäure erhält.

d) Eine Lösung von 21,3 g der gemäss c) erhältlichen Verbindung und 160 g Polyphosphorsäureester in 150 ml Chloroform wird 45 Minuten lang unter Rückfluss erhitzt. Man lässt abkühlen und gibt zum Reaktionsgemisch 200 ml 0,5 N Natronlauge. Man trennt die organische Phase ab und extrahiert die wässrige Phase mit 100 ml Chloroform. Man trocknet die vereinigten organischen Extrakte mit Natriumsulfat, filtriert, engt ein und chromatographiert den Rückstand ("Flash") mit einem Essigester-Hexan-8:92-Gemisch, worauf man 6-Methoxy-8-methyl-2H-[1]benzothiopyran-4-on erhält.

Beispiel 17:

a) Herstellung von 10000 Tabletten mit 10 mg Wirkstoff:

Bestandteile:

[4R*,4aS*,10bR*]-7-Brom-4-ethylthiomethyl-1,3,4,4a,5,10b-hexahydro-9-methoxy-2-methyl-2H-[1]benzopyrano[4,3-c]pyridin-hydrochlorid 100,00 g
Lactose 2400,00 g
Maisstärke 125,00 g
Polyethylenglycol 6000 150,00 g
Magnesiumstearat 40,00 g
Wasser (steril) q.s.

Das pulverige Material wird durch ein Sieb mit Oeffnungen von 0,6 mm gedrückt. Anschliessend misch man in einem Mischer den Wirkstoff mit der Lactose, dem Magnesiumstearat und der halben Menge Stärke. Die andere halbe Menge Stärke wird in 65 ml Wasser suspendiert und diese Suspension zu einer siedenden Lösung des Polyäthylenglycols 260 ml in Wasser gegeben. Diese gebildete Paste wird mit der Pulvermischung versetzt, welche gegebenenfalls mit weiterem Wasser granuliert wird. Man trocknet das Granulat bei 35°, treibt dieses durch ein Sieb mit 1,2 mm Oeffnungen und komprimiert zu konkaven Tabletten mit oberer Bruchkerbe.

b) Herstellung von 1000 Kapseln mit 10,0 mg Wirkstoff:

Bestandteile:
[4R*,4aS*,10bR*]-7-Brom-4-äthylthiomethyl-1,3,4,4a,5,10b-hexahydro-9-methoxy-2-methyl-2H-[1]benzopyrano[4,3-c]pyridin-hydrochlorid 10,00g
Lactose 207,00 g
Stärke 80,00 g
Magnesiumstearat 3,00 g

Das pulverige Material wird durch ein Sieb mit Oeffnungen von 0,6 mm Durchmesser gedrückt. Anschliessend mischt man in einem Mischer den Wirkstoff mit der Lactose, dem Magnesiumstearat und der Stärke bis man eine homogene Masse erhält. Man füllt Nr. 2 Hartgelatinekapseln mit 300 mg der vorbereiteten Mischung.

Analog kann man Kapseln 5 - 100 mg eines anderen von der Formel I definierten Wirkstoffs herstellen.

## Patentansprüche

Patentansprüche (für benannte Vertragsstaaten BE, CH, DE, FR, GB, IT, LI, LU, NL und SE):

1. Verbindungen der Formel

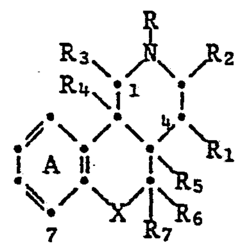

(I),

worin X Sauerstoff oder Schwefel bedeutet, der Ring A unsubstituiert oder durch Hydroxy, veräthertes Hydroxy, Acyloxy, Halogen, Niederalkyl, Arylniederalkyl oder Trifluormethyl ein- bis dreifach oder durch Alkylendioxy an zwei nachbarständigen C-Atomen substituiert sein kann, R Wasserstoff, Niederalkyl, Niederalkenyl, Niederalkinyl, Aroylniederalkyl oder Arylniederalkyl, $R_1$ Wasserstoff, Niederalkyl, Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl, $R_2$ - $R_5$ Wasserstoff oder Niederalkyl und $R_6$ und $R_7$ Wasserstoff bedeuten oder Niederalkyl bedeuten mit der Massgabe, dass $R_1$ Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl bedeuten, und Salze, insbesondere pharmazeutisch annehmbare Salze, dieser Verbindungen.

2. Verbindungen gemäss Anspruch 1 der Formel I, worin X Sauerstoff bedeutet.

3. Verbindungen gemäss Anspruch 1 oder 2 der Formel I, worin $R_2$- $R_7$ Wasserstoff bedeutet.

4. Verbindungen gemäss einem der Ansprüche 1-3 der Formel I, worin X Sauerstoff oder Schwefel bedeutet, der Ring A unsubstituiert oder durch Hydroxy, Acyloxy, veräthertes Hydroxy, Arylniederalkyl, Niederalkyl oder Halogen ein- bis dreifach oder durch Alkylendioxy an zwei nachbarständigen C-Atomen substituiert sein kann, R Wasserstoff oder Niederalkyl, $R_1$ Wasserstoff, Niederalkyl, Niederalkylthio-niederalkyl oder Niederalkoxyniederalkyl und $R_2$ - $R_7$ Wasserstoff bedeuten, und pharmazeutisch annehmbare Salze dieser Verbindungen.

5. Verbindungen gemäss einem der Ansprüche 1-3 der Formel I, worin der Ring A unsubstituiert ist oder durch Niederalkyl, Aryl-niederalkyl, Niederalkoxy, Aryl-niederalkoxy oder Halogen ein- bis dreifach substituiert sein kann, X Sauerstoff oder Schwefel, R Wasserstoff oder Niederalkyl, $R_1$ Wasserstoff, Niederalkyl, Niederalkylthioniederalkyl oder Niederalkoxymethyl und $R_2$ - $R_7$ Wasserstoff bedeuten, und pharmazeutisch annehmbare Salze dieser Verbindungen.

6. Verbindungen gemäss einem der Ansprüche 1-3 der Formel I, worin X Sauerstoff oder Schwefel bedeutet, der Ring A durch $C_1$-$C_4$-Alkoxy, Benzyloxy, $C_1$-$C_4$-Alkyl oder Halogen ein- bis dreifach substituiert sein kann, R $C_1$-$C_4$-Alkyl, $R_1$ Wasserstoff, $C_1$-$C_4$-Alkyl, $C_1$-$C_4$-Alkylthiomethyl oder $C_1$-$C_4$-Alkoxymethyl und $R_2$ - $R_7$ Wasserstoff bedeuten, und pharmazeutisch annehmbare Salze dieser Verbindungen.

7. Verbindungen gemäss einem der Ansprüche 1-6 der Formel

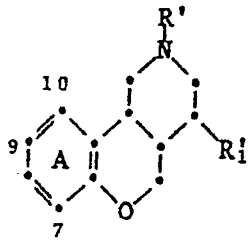

(II),

vorzugsweise mit trans-4a,10b-Ringverknüpfung, worin der Ring A vorzugsweise in 9- oder 10-Stellung durch $C_1$-$C_4$-Alkoxy und in 7-Stellung durch $C_1$-$C_4$-Alkyl oder Halogen sustituiert ist, R' $C_1$-$C_4$-Alkyl und $R_1^!$ Wasserstoff oder $C_1$-$C_4$-Alkylthiomethyl bedeuten, und pharmazeutisch annehmbare Salze dieser Verbindungen.

8. Verbindungen gemäss Anspruch 7 der Formel II, worin worin trans-4a,10b-Ringverknüpfung vorliegt und der Ring A in 7-Stellung durch $C_1$-$C_3$-Alkyl, Brom oder Chlor und in 9-Stellung durch $C_1$-$C_3$-Alkoxy substituiert ist, R' $C_1$-$C_3$-Alkyl und $R_1^!$ Wasserstoff oder $C_1$-$C_3$-Alkylthiomethyl bedeuten, und pharmazeutisch annehmbare Salze davon.

9. [4R*,4aS*,10bR*]-7-Brom-4-äthylthiomethyl-1,3,4,4a,6,10b-hexahydro-9-methoxy-2-methyl-2H-[1]benzopyrano[4,3-c]pyridin gemäss Anspruch 7 oder ein pharmazeutisch annehmbares Salz davon.

10. [4aR*,10bS*]-1,3,4,4a,5,10b-Hexahydro-9-methoxy-2,7-dimethyl-2H[1]benzopyrano[4,3-c]pyridin gemäss Anspruch 7 oder ein pharmazeutisch annehmbares Salz davon.

11. Die in den Ansprüchen 1-10 beanspruchten Verbindungen zur Anwendung in einem Verfahren zur Behandlung des menschlichen oder tierischen Körpers.

12. Die in den Ansprüchen 1-10 beanspruchten Verbindungen zur Anwendung in einem Verfahren zur Behandlung des menschlichen oder tierischen Körpers von psychotropen Störungen.

13. Die in den Ansprüchen 1-10 beanspruchten Verbindungen als Serotonin-2-Rezeptor-Antagonisten.

14. Pharmazeutische Präparate enthaltend Verbindungen der Ansprüche 1 bis 11 oder ein pharmazeutisch annehmbares Salz davon zusammen mit einem pharmauzeutisch geeigneten Trägermaterial.

15. Verwendung der in den Ansprüchen 1 bis 10 genannten Verbindungen zur Herstellungen von pharmazeutischen Präparaten.

16. Verfahren zur Herstellung von den in Anspruch 1 genannten Verbindungen der Formel I, in der alle Symbole die in Anspruch 1 angegebenen Bedeutungen haben, und deren Salzen, gekennzeichnet durch

a) Reduktion einer Verbindung der Formel:

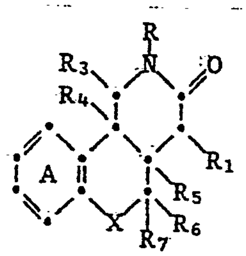

(III),

worin der Ring A, X, R, $R_1$ und $R_3$ - $R_7$ die weiter vorn genannten Bedeutungen haben,

b) durch Reduktion einer ungesättigten Verbindung, welche den Verbindungen der Formel I entspricht, welche aber über eine zusätzliche C-C-Doppelbindung innerhalb von einem oder von beiden heterocyclischen Ringsystemen oder über eine zusätzliche Kohlenstoff-Stickstoff-Doppelbindung (Imin- oder Iminiumbindung) verfügt;

c) durch Zyklisieren einer Verbindung der Formel IV oder IVa

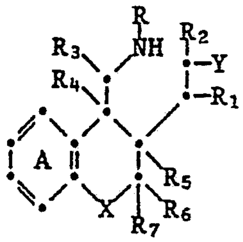

(IV)

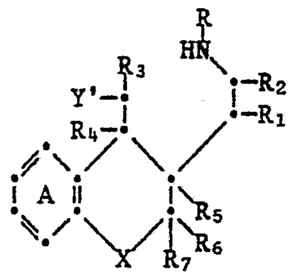

(IVa),

worin der Ring A, X, R und $R_1$ - $R_7$ die genannten Bedeutungen haben und Y und Y' verestertes Hydroxy darstellen;

d) durch Reduktion einer Verbindung der Formel

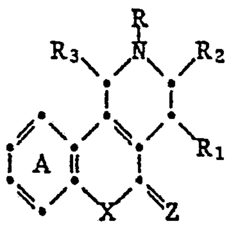

(V)

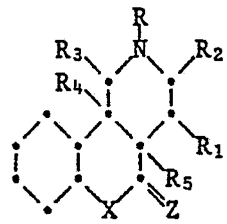

(Va),

worin in beiden Formeln Z Sauerstoff bedeutet, und X, R und $R_1$ - $R_3$ (Formel V) oder X, R und $R_1$ - $R_5$ (Formel Va) wie weiter vorn definiert sind;

e) zur Herstellung einer Verbindung der Formel I, worin $R_6$ und $R_7$ dieselben Bedeutungen haben und Niederalkyl darstellen, eine Verbindung der Formel V oder Va mit einer Metall-Niederalkyl-Verbindung kondensiert und gewünschtenfalls eine eventuelle C-C-Doppelbindung an der Ringverknüpfungsstelle reduziert;

f) zur Herstellung einer Verbindung der Formel I, worin $R_1$ - $R_5$ Wasserstoff sind, die Pyridin- oder Pyridiniumgruppe (wenn R eine andere Bedeutung als Wasserstoff hat) in einer Verbindung der Formel

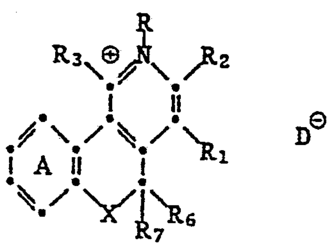

(VI),

reduziert, worin der Ring A, X, $R_1$ - $R_3$ $R_6$ und $R_7$ die genannten Bedeutungen haben, und D− das Anion einer organischen oder anorganischen Säure ist;

g) eine Verbindung der Formel

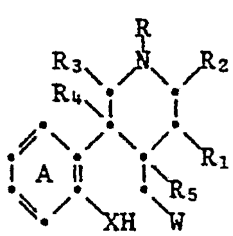

(VII),

worin der Ring A, X, R und $R_1$ - $R_5$ die genannten Bedeutungen haben. und W verestertes Hydroxy darstellt, zyklisiert;

h) zur Herstellung einer Verbindung der Formel I, worin $R_4$ und $R_5$ Wasserstoff sind, eine

Verbindung der Formel

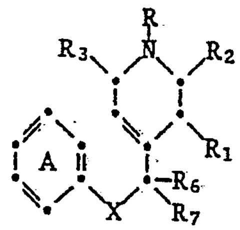

(VIII),

worin der Ring A, X, R, $R_1$ - $R_4$, $R_6$ und $R_7$ die genannten Bedeutungen haben, zyklisiert:

i) zur Herstellung einer Verbindung der Formel I, worin $R_2$ und $R_3$ Wasserstoff sind, eine Verbindung der Formel

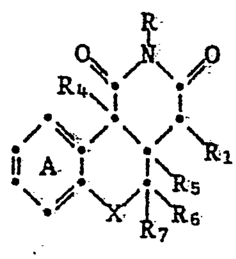

(IX),

worin X, R und $R_1$ und $R_4$ - $R_7$ die weiter vorn genannten Bedeutungen haben oder eine Verbindung der Formel

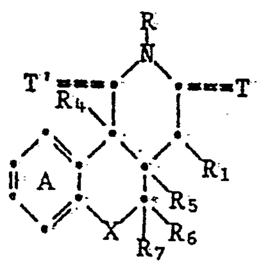

(IXa),

worin T Sauerstoff bedeutet, wenn T' die Bedeutung von zwei Wasserstoffatomen oder von einem Wasserstoffatom und einer Niederalkylgruppe hat, oder worin T' Sauerstoff bedeutet, wenn T die Bedeutung von zwei Wasserstoffatomen oder von einem Wasserstoffatom und einer Niederalkylgruppe hat, und X, R, $R_1$ und $R_4$ - $R_7$ wie oben definiert sind, reduziert;

j) zur Herstellung einer Verbindung der Formel I, worin R Niederalkyl oder Arylniederalkyl bedeutet, eine Verbindung der Formel

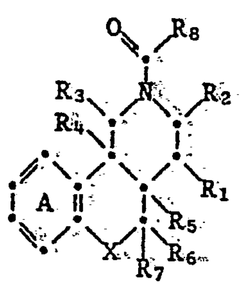

(X),

worin der Ring A, X und $R_1$ - $R_7$ die genannten Bedeutungen haben, $R_8$ Wasserstoff oder Aryl oder Niederalkyl oder Arylniederalkyl mit einer um ein C-Atom verkürzten Kettenlänge bedeutet, reduziert;

k) zur Herstellung einer Verbindung der Formel I, worin $R_4$ - $R_7$ Wasserstoff sind, eine Verbindung der Formel

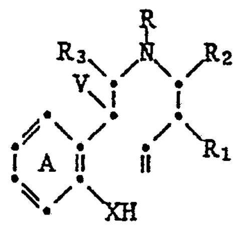

(XI),

worin der Ring A, X, R und $R_1$ - $R_3$ die genannten Bedeutungen haben und V verestertes Hydroxy bedeutet. zyklisiert;

1) eine Verbindung der Formel

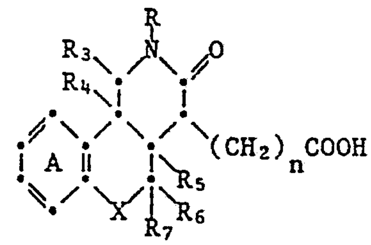

(XII),

oder ein reaktives, funktionelles Derivat davon, z.B. einen Niederalkylester und worin n die Werte null oder eins bis sechs hat und der Ring A, X, R und $R_3$ bis $R_7$ die genannten Bedeutungen haben, durch Decarboxylierung und Reduktion oder durch Reduktion und Umwandlung der Seitenkette derivatisiert, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in einzelne Isomere oder Racemate auftrennt und/oder ein Racemat in die optischen Antipoden aufspaltet.

17. Die nach dem Verfahren gemäss Anspruch 16 erhältlichen Verbindungen.

Patentansprüche (für die benannten Vertragsstaaten AT, ES und GR):

1. Verfahren zur Herstellung von Verbindungen der Formel

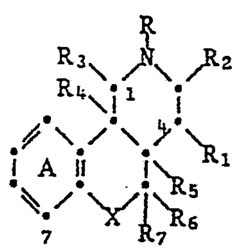

(I),

worin X Sauerstoff oder Schwefel bedeutet, der Ring A unsubstituiert oder durch Hydroxy, veräthertes Hydroxy, Acyloxy, Halogen, Niederalkyl, Arylniederalkyl oder Trifluormethyl ein- bis dreifach oder durch Alkylendioxy an zwei nachbarständigen C-Atomen substituiert sein kann, R Wasserstoff, Niederalkyl, Niederalkenyl, Niederalkinyl, Aroylniederalkyl oder Arylniederalkyl, $R_1$ Wasserstoff, Niederalkyl, Niederalkylthio-niederalkyl, Arylniederalkylthio-niederalkyl, Aminoniederalkyl, Niederalkylaminoniederalkyl, Diniederalkylaminoniederalkyl. Acylaminoniederalkyl, Hydroxyniederalkyl, Acyloxyniederalkyl, veräthertes Hydroxyniederalkyl oder Cyanniederalkyl, $R_2$ - $R_5$ Wasserstoff oder Niederalkyl und $R_6$ und $R_7$ Wasserstoff bedeuten oder Niederalkyl bedeuten mit der Massgabe, dass $R_1$ Niederalkylthio-niederalkyl, Arylniederalkylthio-niederalkyl, Aminoniederalkyl, Niederalkylaminoniederalkyl, Diniederalkylaminoniederalkyl, Acylaminoniederalkyl, Hydroxyniederalkyl, Acyloxyniederalkyl, veräthertes Hydroxyniederalkyl oder Cyanniederalkyl bedeuten, und Salzen, insbesondere pharmazeutisch annehmbaren Salzen, dieser Verbindungen gekennzeichnet durch

a) Reduktion einer Verbindung der Formel:

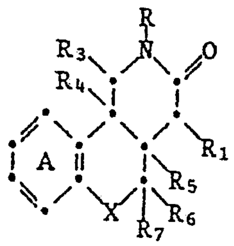

(III),

worin der Ring A, X, R, $R_1$ und $R_3$ - $R_7$ die weiter vorn genannten Bedeutungen haben,

b) durch Reduktion einer ungesättigten Verbindung, welche den Verbindungen der Formel I entspricht, welche aber über eine zusätzliche C-C-Doppelbindung innerhalb von einem oder von beiden heterocyclischen Ringsystemen oder über eine zusätzliche Kohlenstoff-Stickstoff-Doppelbindung (Imin- oder Iminiumbindung) verfügt;

c) durch Zyklisieren einer Verbindung der Formel IV oder IVa

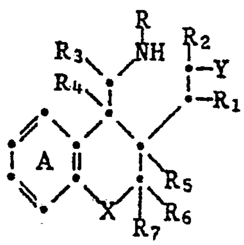

(IV)

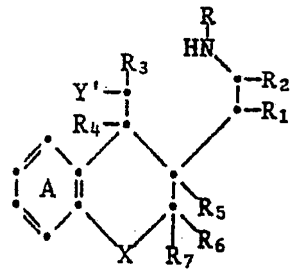

(IVa),

worin der Ring A, X, R und $R_1$ - $R_7$ die genannten Bedeutungen haben und Y und Y' verestertes Hydroxy darstellen;

d) durch Reduktion einer Verbindung der Formel

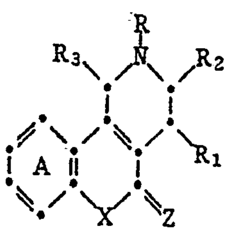

(V)

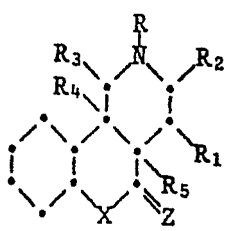

(Va),

worin in beiden Formeln Z Sauerstoff bedeutet, und X, R und $R_1$ - $R_3$ (Formel V) oder X, R und $R_1$ - $R_5$ (Formel Va) wie weiter vorn definiert sind;

e) zur Herstellung einer Verbindung der Formel I, worin $R_6$ und $R_7$ dieselben Bedeutungen haben und Niederalkyl darstellen, eine Verbindung der Formel V oder Va mit einer Metall-Niederalkyl-Verbindung kondensiert und gewünschtenfalls eine eventuelle C-C-Doppelbindung an der Ringverknüpfungsstelle reduziert;

f) zur Herstellung einer Verbindung der Formel I, worin $R_1$ - $R_5$ Wasserstoff sind, die Pyridin- oder Pyridiniumgruppe (wenn R eine andere Bedeutung als Wasserstoff hat) in einer Verbindung der Formel

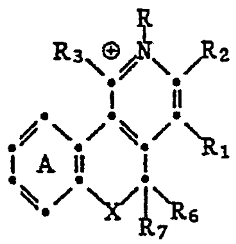 $D^{\ominus}$ (VI),

reduziert, worin der Ring A, X, $R_1$ - $R_3$ $R_6$ und $R_7$ die genannten Bedeutungen haben, und D- das Anion einer organischen oder anorganischen Säure ist;

g) eine Verbindung der Formel

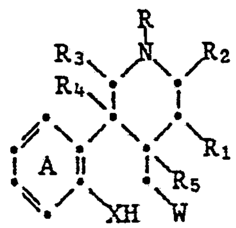 (VII),

worin der Ring A, X, R und $R_1$ - $R_5$ die genannten Bedeutungen haben, und W verestertes Hydroxy darstellt, zyklisiert;

h) zur Herstellung einer Verbindung der Formel I, worin $R_4$ und $R_5$ Wasserstoff sind, eine Verbindung der Formel

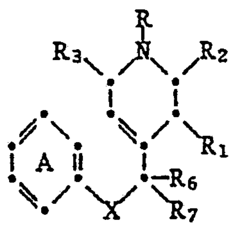 (VIII),

worin der Ring A, X, R, $R_1$ - $R_4$, $R_6$ und $R_7$ die genannten Bedeutungen haben, zyklisiert;

i) zur Herstellung einer Verbindung der Formel I, worin $R_2$ und $R_3$ Wasserstoff sind, eine Verbindung der Formel

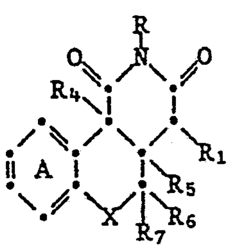 (IX),

worin X, R und $R_1$ und $R_4$ - $R_7$ die weiter vorn genannten Bedeutungen haben oder eine Verbindung der Formel

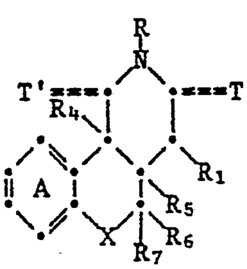 (IXa),

worin T Sauerstoff bedeutet, wenn T' die Bedeutung von zwei Wasserstoffatomen oder von einem Wasserstoffatom und einer Niederalkylgruppe hat, oder worin T' Sauerstoff bedeutet, wenn T die Bedeutung von zwei Wasserstoffatomen oder von einem Wasserstoffatom und einer Niederalkylgruppe hat, und X, R, $R_1$ und $R_4$ - $R_7$ wie oben definiert sind, reduziert;

j) zur Herstellung einer Verbindung der Formel I, worin R Niederalkyl oder Arylniederalkyl bedeutet, eine Verbindung der Formel

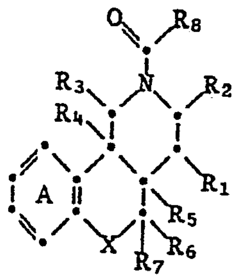
(X),

worin der Ring A, X und $R_1$ - $R_7$ die genannten Bedeutungen haben, $R_8$ Wasserstoff oder Aryl oder Niederalkyl oder Arylniederalkyl mit einer um ein C-Atom verkürzten Kettenlänge bedeutet, reduziert;

k) zur Herstellung einer Verbindung der Formel I, worin $R_4$ - $R_7$ Wasserstoff sind, eine Verbindung der Formel

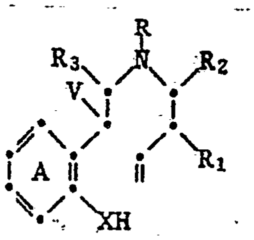
(XI),

worin der Ring A, X, R und $R_1$ - $R_3$ die genannten Bedeutungen haben und V verestertes Hydroxy bedeutet, zyklisiert;

l) eine Verbindung der Formel

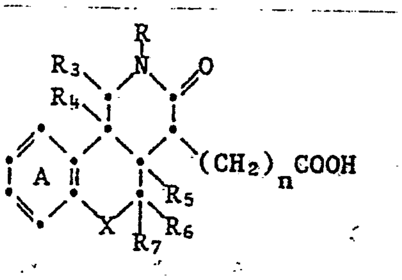
(XII),

oder ein reaktives, funktionelles Derivat davon, z.B. einen Niederalkylester und worin n die Werte null oder eins bis sechs hat und der Ring A, X, R und $R_3$ bis $R_7$ die genannten Bedeutungen haben, durch Decarboxylierung und Reduktion oder durch Reduktion und Umwandlung der Seitenkette derivatisiert, und gewünschtenfalls reaktive funktionelle Gruppen intermediär bei der Ausführung eines der genannten Verfahren schützt und anschliessend die freie Verbindung der Formel I isoliert und/oder eine erhältliche Verbindung der Formel I in eine andere Verbindung der Formel I umwandelt und/oder eine erhältliche freie Verbindung in ein Salz oder ein Salz in die freie Verbindung oder in ein anderes Salz umwandelt und/oder ein erhältliches Gemisch von Isomeren oder Racematen in einzelne Isomere oder Racemate auftrennt und/oder ein Racemat in die optischen Antipoden aufspaltet.

2. Verfahren zur Herstellung von Verbindungen gemäss Anspruch 1 der Formel I, worin X Sauerstoff bedeutet.

3. Verfahren zur Herstellung von Verbindungen gemäss Anspruch 1 oder 2 der Formel I, worin $R_2$ -$R_7$ Wasserstoff bedeutet.

4. Verfahren zur Herstellung von Verbindungen gemäss einem der Ansprüche 1-3 der Formel I, worin X Sauerstoff oder Schwefel bedeutet, der Ring A unsubstituiert oder durch Hydroxy, Acyloxy, veräthertes Hydroxy, Arylniederalkyl, Niederalkyl oder Halogen ein-bis dreifach oder durch Alkylendioxy an zwei nachbarständigen C-Atomen substituiert sein kann, R Wasserstoff oder Niederalkyl, $R_1$ Wasserstoff, Niederalkyl, Niederalkylthio-niederalkyl oder Niederalkoxyniederalkyl und $R_2$ - $R_7$ Wasserstoff bedeuten,

und pharmazeutisch annehmbare Salze dieser Verbindungen.

5. Verfahren zur Herstellung von Verbindungen gemäss einem der Ansprüche 1-3 der Formel I, worin der Ring A unsubstituiert ist oder durch Niederalkyl, Aryl-niederalkyl, Niederalkoxy, Aryl-niederalkoxy oder Halogen ein- bis dreifach substituiert sein kann, X Sauerstoff oder Schwefel, R Wasserstoff oder Niederalkyl, $R_1$ Wasserstoff, Niederalkyl, Niederalkylthioniederalkyl oder Niederalkoxymethyl und $R_2$ - $R_7$ Wasserstoff bedeuten, und pharmazeutisch annehmbare Salze dieser Verbindungen.

6. Verfahren zur Herstellung von Verbindungen gemäss einem der Ansprüche 1-3 der Formel I, worin X Sauerstoff oder Schwefel bedeutet, der Ring A durch $C_1$-$C_4$-Alkoxy, Benzyloxy, $C_1$-$C_4$-Alkyl oder Halogen ein- bis dreifach substituiert sein kann, R $C_1$-$C_4$-Alkyl, $R_1$ Wasserstoff, $C_1$-$C_4$-Alkyl, $C_1$-$C_4$-Alkylthiomethyl oder $C_1$-$C_4$-Alkoxymethyl und $R_2$ - $R_7$ Wasserstoff bedeuten, und pharmazeutisch annehmbare Salze dieser Verbindungen.

7. Verfahren zur Herstellung von Verbindungen gemäss einem der Ansprüche 1-6 der Formel I,

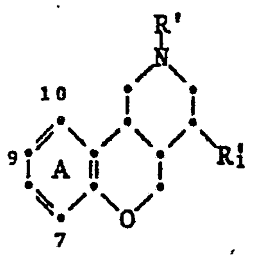

(II),

vorzugsweise mit trans-4a,10b-Ringverknüpfung, worin der Ring A vorzugsweise in 9- oder 10-Stellung durch $C_1$-$C_4$-Alkoxy und in 7-Stellung durch $C_1$-$C_4$-Alkyl oder Halogen substituiert ist, R' $C_1$-$C_4$-Alkyl und $R_1^{!}$ Wasserstoff oder $C_1$-$C_4$-Alkylthiomethyl bedeuten, und pharmazeutisch annehmbare Salze dieser Verbindungen.

8. Verfahren zur Herstellung von Verbindungen gemäss Anspruch 7 der Formel II, worin trans-4a,10b-Ringverknüpfung vorliegt und der Ring A in 7-Stellung durch $C_1$-$C_3$-Alkyl, Brom oder Chlor und in 9-Stellung durch $C_1$-$C_3$-Alkoxy substituiert it, R' $C_1$-$C_3$-Alkyl und $R_1^{!}$ Wasserstoff oder $C_1$-$C_3$-Alkylthiomethyl bedeuten, und pharmazeutisch annehmbare Salze davon.

9. Verfahren zur Herstellung von [4R*,4aS*,10bR*]-7-Brom-4-äthylthiomethyl-1,3,4,4a,5,10b-hexahydro-9-methoxy-2-methyl-2H-[1]benzopyrano[4,3-c]pyridin gemäss Anspruch 1 oder von einem pharmazeutisch annehmbaren Salz davon.

10. Verfahren zur Herstellung von [4aR*,10bS*]-1,3,4,4a,5,10b-Hexahydro-9-methoxy-2,7-dimethyl-2H[1]benzopyrano[4,3-c]pyridin oder von einem pharmazeutisch annehmbare Salz davon.

11. Verfahren zur Herstellung von pharmazeutischen Präparaten, dadurch gekennzeichnet, dass man Verbindungen der Ansprüche 1-10 oder ein pharmazeutisch annehmbares Salz davon zusammen mit einem pharmazeutisch geeigneten Trägermaterial vermischt.

Europäisches Patentamt

# EUROPÄISCHER RECHERCHENBERICHT

Nummer der Anmeldung

EP 86810496.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | EP - A2 - 0 161 218 (CIBA-GEIGY AG)<br>* Gesamt *<br>-- | 1-17 | C 07 D 493/04<br>C 07 D 491/052<br>C 07 D 493/14<br>C 07 D 491/153<br>A 61 K 31/44<br>//(C 07 D 493/04<br>C 07 D 311:00<br>C 07 D 221:00)<br>(C 07 D 491/052<br>C 07 D 335:00<br>C 07 D 221:00) |
| A | CHEMICAL ABSTRACTS, Band 100, Nr. 25, 18. Juni 1984, Columbus, Ohio, USA<br>ROWLAND, D.A.; GILLEPSIE, R.J.; AGER, I.R.; CLEMENTS-JEWERY, ST.; GARNER, C.R.; "Benzothiopyrano-pyridinones and their salts, their use us midicaments, and compositions containing them"<br>Seite 595, Spalte 1, Zusammen-fassung Nr. 209 782j<br>& EP 0 099 303, 25. Jänner 1984<br>-- | 1,12 | |
| | | | RECHERCHIERTE SACHGEBIETE (Int. Cl.4) |
| A,P | CHEMICAL ABSTRACTS, Band 104, Nr. 13, 31. März 1986, Columbus, Ohio, USA<br>HORN, A.S.; "Naphthoxazines"<br>Seite 730, Spalte 2, Zusammen-fassung Nr. 109 662j<br>& US 4 540 691, 10. September 1985<br>---- | 1,12 | C 07 D 493/00<br>C 07 D 491/00 |

Der vorliegende Recherchenbericht wurde für alle Patentansprüche erstellt.

| Recherchenort | Abschlußdatum der Recherche | Prüfer |
|---|---|---|
| WIEN | 13-02-1987 | BRUS |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82